# EUROPÄISCHE PATENTANMELDUNG

(11) **EP 2 725 020 A1**
(43) Veröffentlichungstag der Anmeldung: **30.04.2014**
(21) Anmeldenummer: 14151109.7
(22) Anmeldetag: 04.06.2009
(51) Int. Cl.: C07D 401/04, C07D 401/14, C07D 417/04, C07D 417/14, A01N 43/78, A01N 43/82, A01N 43/56, A01N 43/88

(54) **Neue heteroaromatische Amide und Thioamide als Schädlingsbekämpfungsmittel**

(30) Priorität: 13.06.2008 EP 08158247
(62) Teilanmeldung aus: 09761421.8
(71) Anmelder: Bayer CropScience AG, 40789 Monheim am Rhein (DE)
(72) Erfinder: Die Erfindernennung liegt noch nicht vor
(74) Vertreter: BIP Patents

(57) **Zusammenfassung**

Die vorliegende Anmeldung betrifft neue Amide und Thioamide der Formel (I) 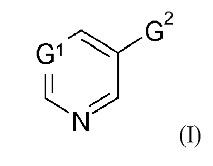 worin
G¹ für N, CH oder C-Halogen steht, und
G² für 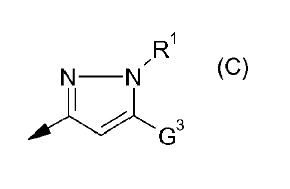 steht,
worin
R¹ für Wasserstoff oder Alkyl steht und
G³ für C(=X)NR²R³ steht,
worin
X für Sauerstoff oder Schwefel steht,
Verfahren zu Ihrer Herstellung und ihre Verwendung zur Bekämpfung von tierischen Schädlingen, vor allem von Arthropoden und insbesondere von Insekten.

## Beschreibung

Die vorliegende Anmeldung betrifft neue Amide und Thioamide, Verfahren zu Ihrer Herstellung und ihre Verwendung zur Bekämpfung von tierischen Schädlingen, zu denen Arthropoden und insbesondere Insekten zählen.

Bestimmte Amide sind bereits als insektizid wirksame Verbindungen bekannt geworden (vgl. DE 2221647).

Moderne Planzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch neue Verbindungen der Formel (I), 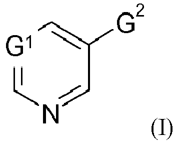 worin
(Ia)
   - G¹: für N oder C-Halogen steht, und
   - G²: für einen Rest aus der Reihe 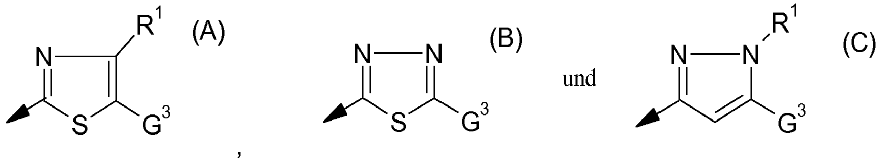
   steht,
   worin
   - R¹: für Wasserstoff oder Alkyl steht und
   - G³: für C(=X)NR²R³ steht,
   worin
   - X: für Sauerstoff oder Schwefel steht,
   - R²: für einen Rest aus der Reihe Wasserstoff, Alkyl, Halogenalkyl, Alkoxy, Halogenalkoxy, Alkenyl, Alkoxyalkyl, gegebenenfalls durch Halogen substituiertes Alkylcarbonyl, gegebenenfalls durch Halogen substituiertes Alkoxycarbonyl und gegebenenfalls durch Halogen substituiertes Cycloalkylcarbonyl steht,
   - und R³: für einen Rest aus der Reihe Wasserstoff, Alkyl, Halogenalkyl, Cyanoalkyl, Alkenyl, Halogenalkenyl, Alkinyl, Halogenalkinyl, Alkoxy, Halogenalkoxy, gegebenenfalls durch Halogen substituiertes Alkoxyalkyl, gegebenenfalls durch Halogen substituiertes Bis(alkoxy)alkyl, gegebenenfalls durch Halogen substituiertes Alkylthioalkyl, gegebenenfalls durch Halogen substituiertes Alkylcarbonylalkyl, gegebenenfalls durch Halogen substituiertes Alkylsulfinylalkyl, gegebenenfalls durch Halogen substituiertes Alkylsulfonylalkyl, gegebenenfalls durch Halogen substituiertes Alkoxycarbonylalkyl, Alkinyloxy, Alkinyloxycarbonyl, gegebenenfalls substituiertes Cycloalkyl, gegebenenfalls substituiertes Cycloalkylcarbonyl, gegebenenfalls substituiertes Cycloalkylalkyl, gegebenenfalls substituiertes Heterocyclyl, gegebenenfalls substituiertes Heterocyclylalkyl, gegebenenfalls substituiertes Arylalkyl, gegebenenfalls substituiertes Hetarylalkyl und NR⁴R⁵ steht, worin R⁴ und R⁵ unabhängig voneinander für einen Rest aus der Reihe Wasserstoff, Alkyl, Halogenalkyl, Cycloalkyl, Alkoxy, Alkylcarbonyl, Alkoxycarbonyl, Hetaryl und Heterocyclyl stehen oder R⁴ und R⁵ gemeinsam mit dem Stickstoffatom, an das sie gebunden sind, einen gegebenenfalls substituierten Hetrocyclus bilden,
   oder R² und R³ gemeinsam mit dem Stickstoffatom, an das sie gebunden sind, einen gegebenenfalls substituierten und gegebenenfalls weitere Heteroatome enthaltenden Ring bilden, oder (Ib) worin G¹ für CH steht und G² für 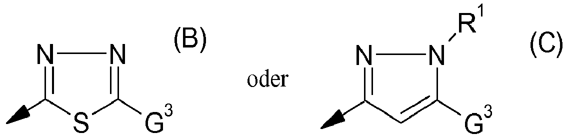 steht, worin R¹ für Wasserstoff oder Alkyl steht und G³ für C(=X)NR²R³ steht, worin X für Sauerstoff oder Schwefel steht, R² die oben angegebenen Bedeutungen hat und R³ die oben angegebenen Bedeutungen hat oder R² und R³ gemeinsam mit dem Stickstoffatom, an das sie gebunden sind, einen gegebenenfalls substituierten und gegebenenfalls weitere Heteroatome enthaltenden Ring bilden oder (Ic) worin G¹ für CH steht, G² für 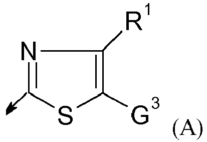 steht R¹ für Wasserstoff oder Alkyl steht und G³ für C(=S)NR²R³ steht, worin R² die oben angegebenen Bedeutungen hat und R³ die oben angegebenen Bedeutungen hat oder R² und R³ gemeinsam mit dem Stickstoffatom, an das sie gebunden sind, einen gegebenenfalls substituierten und gegebenenfalls weitere Heteroatome enthaltenden Ring bilden oder (Id) worin G¹ für CH steht und G² für 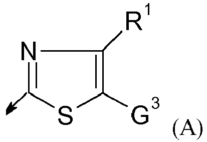 steht,
   - R¹: für Wasserstoff oder Alkyl steht und
   - G³: für C(=O)NR²R³ steht,
   worin R² für einen Rest aus der Reihe Wasserstoff, Alkyl, Halogenalkyl, Alkoxy, Halogenalkoxy, Alkenyl, Alkoxyalkyl, gegebenenfalls durch Halogen substituiertes Alkylcarbonyl, gegebenenfalls durch Halogen substituiertes Alkoxycarbonyl und gegebenenfalls durch Halogen substituiertes Cycloalkylcarbonyl steht, und R³ für eine Rest aus der Reihe Halogenalkyl, Cyanoalkyl, Halogenalkenyl, Halogenalkinyl, Alkoxy, Halogenalkoxy, durch Halogen substituiertes Alkoxyalkyl, gegebenenfalls durch Halogen substituiertes Bis(alkoxy)alkyl, gegebenenfalls durch Halogen substituiertes Alkylthioalkyl, gegebenenfalls durch Halogen substituiertes Alkylcarbonylalkyl, gegebenenfalls durch Halogen substituiertes Alkylsulfinylalkyl, gegebenenfalls durch Halogen substituiertes Alkylsulfonylalkyl, gegebenenfalls durch Halogen substituiertes Alkoxycarbonylalkyl, Alkinyloxy, Alkinyloxycarbonyl, durch Halogen, Cyano, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkyl, gegebenenfalls substituiertes Cycloalkylcarbonyl, gegebenenfalls substituiertes Cycloalkylalkyl, gegebenenfalls substituiertes Heterocyclyl, gegebenenfalls substituiertes Heterocyclylalkyl, durch Halogen, Cyano, Alkyl, Halogenalkyl, Alkoxy oder Halogenalkoxy substituiertes Arylalkyl, gegebenenfalls substituiertes Hetarylalkyl und NR⁴R⁵ steht, worin R⁴ und R⁵ unabhängig voneinander für einen Rest aus der Reihe Wasserstoff, Alkyl, Halogenalkyl, Cycloyalkyl, Alkoxy, Alkylcarbonyl, Alkoxycarbonyl, Hetaryl und Heterocyclyl stehen oder R⁴ und R⁵ gemeinsam mit dem Stickstoffatom, an das sie gebunden sind, einen gegebenenfalls substituierten Hetrocyclus bilden oder (Ie) worin G¹ für CH steht und G² für 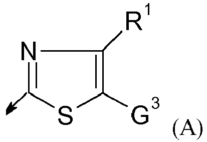 steht, R¹ für Wasserstoff steht und G³ für C(=O)NR²R³ steht, worin R² Wasserstoff oder Alkyl steht und R³ für Wasserstoff oder Alkyl steht.
sowie Salze und N-Oxide der Verbindungen der Formel (I).

Insbesondere gelöst wird die oben genannte Aufgabe, sowie weitere nicht explizit genannte Aufgaben, die aus den hierin diskutierten Zusammenhängen ableitbar oder erschließbar sind, durch neue Verbindungen der oben genannten Formel (I), worin
(Ia)
   - G¹: für N oder C-Halogen steht, und
   - G²: für einen Rest aus der Reihe 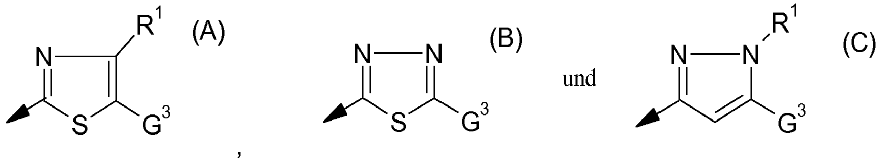 steht, worin R¹ für Wasserstoff oder Alkyl steht und G³ für C(=X)NR²R³ steht, worin X für Sauerstoff oder Schwefel steht, R² für einen Rest aus der Reihe Wasserstoff, Alkyl, Halogenalkyl, Alkoxy, Halogenalkoxy, Alkenyl, Alkoxyalkyl, gegebenenfalls durch Halogen substituiertes Alkylcarbonyl, gegebenenfalls durch Halogen substituiertes Alkoxycarbonyl und gegebenenfalls durch Halogen substituiertes Cycloalkylcarbonyl steht, und R³ für einen Rest aus der Reihe Wasserstoff, Alkyl, Halogenalkyl, Cyanoalkyl, Alkenyl, Halogenalkenyl, Alkinyl, Halogenalkinyl, Alkoxy, Halogenalkoxy, gegebenenfalls durch Halogen substituiertes Alkoxyalkyl, gegebenenfalls durch Halogen substituiertes Bis(alkoxy)alkyl, gegebenenfalls durch Halogen substituiertes Alkylthioalkyl, gegebenenfalls durch Halogen substituiertes Alkylcarbonylalkyl, gegebenenfalls durch Halogen substituiertes Alkylsulfinylalkyl, gegebenenfalls durch Halogen substituiertes Alkylsulfonylalkyl, gegebenenfalls durch Halogen substituiertes Alkoxycarbonylalkyl, Alkinyloxy, Alkinyloxycarbonyl, gegebenenfalls durch Halogen, Cyano, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkyl, gegebenenfalls durch Halogen, Cyano, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylcarbonyl, gegebenenfalls durch Halogen, Cyano, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylalkyl, gegebenenfalls substituiertes Heterocyclyl, gegebenenfalls durch Halogen, Cyano (auch im Alkylteil), Nitro, Alkyl, Halogenalkyl, Cycloalkyl, Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Alkylcycloalkyl, Cycloalkylalkyl, Alkylcarbonyl, Alkoxycarbonyl oder Aminocarbonyl substituiertes Heterocyclylalkyl, gegebenenfalls durch Halogen, Cyano, Alkyl, Halogenalkyl, Alkoxy oder Halogenalkoxy substituiertes Arylalkyl, gegebenenfalls durch Halogen, Cyano (auch im Alkylteil), Nitro, Alkyl, Halogenalkyl, Cycloalkyl, Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Alkylcycloalkyl, Alkylcarbonyl, Alkoxycarbonyl oder Aminocarbonyl substituiertes Hetarylalkyl und NR⁴R⁵ steht, worin R⁴ und R⁵ unabhängig voneinander für einen Rest aus der Reihe Wasserstoff, Alkyl, Halogenalkyl, Cycloyalkyl, Alkoxy, Alkylcarbonyl, Alkoxycarbonyl, Hetaryl und Heterocyclyl stehen oder R⁴ und R⁵ gemeinsam mit dem Stickstoffatom, an das sie gebunden sind, einen gegebenenfalls substituierten Hetrocyclus bilden oder R² und R³ gemeinsam mit dem Stickstoffatom, an das sie gebunden sind, einen gegebenenfalls substituierten und gegebenenfalls weitere Heteroatome enthaltenden Ring bilden, oder (Ib) worin G¹ für CH steht und G² für 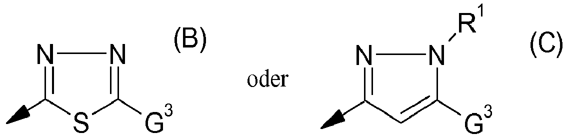 steht, worin R¹ für Wasserstoff oder Alkyl steht und G³ für C(=X)NR²R³ steht, worin X für Sauerstoff oder Schwefel steht, R² die oben angegebenen Bedeutungen hat und R³ die oben angegebenen Bedeutungen hat oder R² und R³ gemeinsam mit dem Stickstoffatom, an das sie gebunden sind, einen gegebenenfalls substituierten und gegebenenfalls weitere Heteroatome enthaltenden Ring bilden oder (Ic) worin G¹ für CH steht, G² für 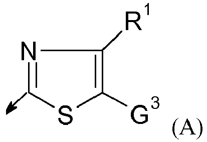 steht R¹ für Wasserstoff oder Alkyl steht und G³ für C(=S)NR²R³ steht, worin R² die oben angegebenen Bedeutungen hat und R³ die oben angegebenen Bedeutungen hat oder R² und R³ gemeinsam mit dem Stickstoffatom, an das sie gebunden sind, einen gegebenenfalls substituierten und gegebenenfalls weitere Heteroatome enthaltenden Ring bilden oder (Id) worin G¹ für CH steht und G² für 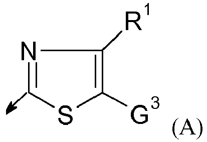 steht, R¹ für Wasserstoff oder Alkyl steht und G³ für C(=O)NR²R³ steht, worin R² für einen Rest aus der Reihe Wasserstoff, Alkyl, Halogenalkyl, Alkoxy, Halogenalkoxy, Alkenyl, Alkoxyalkyl, gegebenenfalls durch Halogen substituiertes Alkylcarbonyl, gegebenenfalls durch Halogen substituiertes Alkoxycarbonyl und gegebenenfalls durch Halogen substituiertes Cycloalkylcarbonyl steht, und R³ für einen Rest aus der Reihe Halogenalkyl, Cyanoalkyl, Halogenalkenyl, Halogenalkinyl, Alkoxy, Halogenalkoxy, durch Halogen substituiertes Alkoxyalkyl, gegebenenfalls durch Halogen substituiertes Bis(alkoxy)alkyl, gegebenenfalls durch Halogen substituiertes Alkylthioalkyl, gegebenenfalls durch Halogen substituiertes Alkylcarbonylalkyl, gegebenenfalls durch Halogen substituiertes Alkylsulfinylalkyl, gegebenenfalls durch Halogen substituiertes Alkylsulfonylalkyl, gegebenenfalls durch Halogen substituiertes Alkoxycarbonylalkyl, Alkinyloxy, Alkinyloxycarbonyl, durch Halogen, Cyano, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkyl, gegebenenfalls durch Halogen, Cyano, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylcarbonyl, gegebenenfalls durch Halogen, Cyano, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylalkyl, gegebenenfalls substituiertes Heterocyclyl, gegebenenfalls durch Halogen, Cyano (auch im Alkylteil), Nitro, Alkyl, Halogenalkyl, Cycloalkyl, Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcycloalkyl, Alkylcarbonyl, Alkoxycarbonyl oder Aminocarbonyl substituiertes Heterocyclylalkyl, durch Halogen, Cyano, Alkyl, Halogenalkyl, Alkoxy oder Halogenalkoxy substituiertes Arylalkyl, gegebenenfalls durch Halogen, Cyano (auch im Alkylteil), Nitro, Alkyl, Halogenalkyl, Cycloalkyl, Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Alkylcycloalkyl, Alkylcarbonyl, Alkoxycarbonyl oder Aminocarbonyl substituiertes Hetarylalkyl und NR⁴R⁵ steht, worin R⁴ und R⁵ unabhängig voneinander für einen Rest aus der Reihe Wasserstoff, Alkyl, Halogenalkyl, Cycloyalkyl, Alkoxy, Alkylcarbonyl, Alkoxycarbonyl, Hetaryl und Heterocyclyl stehen oder R⁴ und R⁵ gemeinsam mit dem Stickstoffatom, an das sie gebunden sind, einen gegebenenfalls substituierten Hetrocyclus bilden oder (Ie) worin G¹ für CH steht und G² für 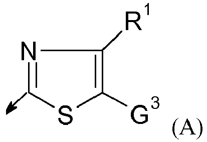 steht, R¹ für Wasserstoff steht und G³ für C(=O)NR²R³ steht, worin R² Wasserstoff oder Alkyl steht und R³ für Wasserstoff oder Alkyl steht.
   sowie Salze und N-Oxide der Verbindungen der Formel (I).
   Weiter wurde gefunden, dass man die neuen Verbindungen der Formel (I), in welcher G² für den Rest (A) und X für Sauerstoff steht, erhält, wenn man
   Verbindungen der Formel (IIa) 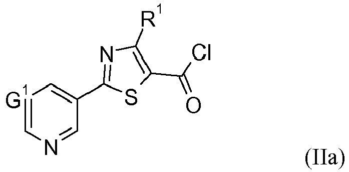 in welcher
   G¹ und R¹ die weiter oben genannten Bedeutungen haben
   mit Verbindungen der Formel (III) 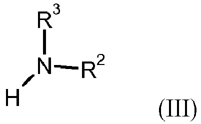 in welcher
   R² und R³ die weiter oben genannten Bedeutungen haben,
   gegebenenfalls in Gegenwart eines geeigneten Verdünnungsmittels und gegebenenfalls in Gegenwart eine Base umsetzt (Verfahren 1),
   dass man ferner die neuen substituierten Verbindungen der Formel (I), in welcher G² für den Rest (B) und X für Sauerstoff steht, erhält, wenn man
   Verbindungen der Formel (IIb) 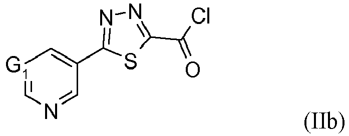 in welcher
   - G¹: die weiter oben genannten Bedeutung hat
   mit Verbindungen der Formel (III) 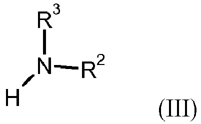 in welcher
   R² und R³ die weiter oben genannten Bedeutungen haben,
   gegebenenfalls in Gegenwart eines geeigneten Verdünnungsmittels und gegebenenfalls in Gegenwart eine Base umsetzt (Verfahren 2),
   und dass man ferner die neuen substituierten Verbindungen der Formel (I), in welcher G² für den Rest (C) und X für Sauerstoff steht, erhält, wenn man
   Verbindungen der Formel (IIc) 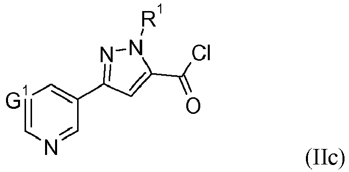 in welcher
   G¹ und R¹ die weiter oben genannten Bedeutung haben
   mit Verbindungen der Formel (III) 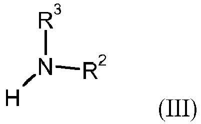 in welcher
   R² und R³ die weiter oben genannten Bedeutungen haben,
   gegebenenfalls in Gegenwart eines geeigneten Verdünnungsmittels und gegebenenfalls in Gegenwart eine Base umsetzt (Verfahren 3).

Die nach diesen Verfahren erhältlichen Verbindungen der Formel (I) lassen sich durch Umsetzung mit einem Schwefelungsreagenz in Verbindungen der Formel (I) überführen, in denen X für Schwefel steht.

Schließlich wurde gefunden, dass die neuen Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die Verbindungen der Formel (I) können gegebenenfalls in Abhängigkeit von der Art der Substituenten als geometrische und/oder als optisch aktive Isomere oder entsprechende Isomerengemische in unterschiedlicher Zusammensetzung vorliegen. Die Erfindung betrifft sowohl die reinen Isomere als auch die Isomerengemische.

Die erfindungsgemäßen Verbindungen können auch als Metallkomplexe vorliegen, wie sie für andere Amide beispielsweise in DE 2221647 beschrieben sind.

Bevorzugte Substituenten bzw. Bereiche der in den oben erwähnten Verbindungen (Ia), (Ib) und (Ic) aufgeführten Reste werden im Folgenden erläutert.
- R¹: steht für Wasserstoff oder C₁-C₆-Alkyl, insbesondere für Wasserstoff oder Methyl.
- R²: steht für Wasserstoff, C₁-C₆-Alkyl, C₁-C₆-Halogenalkyl, C₁-C₄-Alkoxy, C₁-C₄-Halogenalkoxy, C₂-C₄-Alkenyl, C₁-C₄-Alkoxy-C₁-C₄-alkyl, gegebenenfalls durch Halogen substituiertes C₁-C₄-Alkylcarbonyl, gegebenenfalls durch Halogen substituiertes C₁-C₄-Alkoxycarbonyl oder gegebenenfalls durch Halogen substituiertes C₃-C₆-Cycloalkylcarbonyl.
- R³: steht für Wasserstoff, C₁-C₆-Alkyl, C₁-C₆-Halogenalkyl, Cyano-C₁-C₆-alkyl, C₂-C₄-Alkenyl, C₂-C₄-Halogenalkenyl, C₂-C₄-Alkinyl, C₂-C₄-Halogenalkinyl, C₁-C₆-Alkoxy, C₁-C₆-Halogenalkoxy, gegebenenfalls durch Halogen substituiertes C₁-C₆-Alkoxy-C₁-C₆-alkyl, gegebenenfalls durch Halogen substituiertes Bis(C₁-C₆-alkoxy)-C₁-C₆-alkyl, gegebenen-falls durch Halogen substituiertes C₁-C₆-Alkylthio-C₁-C₆-alkyl, gegebenenfalls durch Ha-logen substituiertes C₁-C₄-Alkylcarbonyl-C₁-C₄-alkyl, gegebenenfalls durch Halogen substituiertes C₁-C₆-Alkylsulfmyl-C₁-C₆-alkyl, gegebenenfalls durch Halogen substituiertes C₁-C₆-Alkylsulfonyl-C₁-C₆-alkyl, gegebenenfalls durch Halogen substituiertes C₁-C₆-Alkoxycarbonyl-C₁-C₆-alkyl, für C₂-C₄-Alkinyloxy, für C₂-C₄-Alkinyloxycarbonyl, für gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl, für gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkylcarbonyl, für gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl-C₁-C₆-alkyl, für gegebenenfalls durch Halogen, Cyano (auch im Alkylteil), Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Alkylsulfmyl, C₁-C₆-Alkylsulfonyl, C₁-C₆-Haloalkylsulfinyl, C₁-C₆-Haloalkylsulfonyl, Amino, C₁-C₆-Alkylamino, Di(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₁-C₆-Alkyl-C₃-C₆-cycloalkyl, C₃-C₆-Cycloalkyl-C₁-C₆-alkyl, C₁-C₆-Alkylcarbonyl, C₁-C₆-Alkoxycarbonyl oder Aminocarbonyl substituiertes Heterocyclyl-C₁-C₆-alkyl, für gegebenenfalls durch Halogen, Cyano, C₁-C₆-Alkyl, C₁-C₆-Halogenalkyl, C₁-C₆-Alkoxy oder C₁-C₆-Halogenalkoxy substituiertes Aryl-C₁-C₆-alkyl, für gegebenenfalls durch Halogen, Cyano (auch im Alkylteil), Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₁-C₆-Alkyl-C₃-C₆-cycloalkyl, C₃-C₆-Cycloalkyl-C₁-C₆-alkyl, C₁-C₆-Alkylcarbonyl, C₁-C₆-Alkoxycarbonyl oder Aminocarbonyl substituiertes Hetaryl-C₁-C₆-alkyl, oder für NR⁴R⁵, worin R⁴ und R⁵ unabhängig voneinander für einen Rest aus der Reihe Wasserstoff, C₁-C₆-Alkyl, C₁-C₆-Halogenalkyl, C₃-C₆-Cycloyalkyl, C₁-C₆-Alkoxy, C₁-C₆-Alkylcarbonyl und C₁-C₆-Alkoxycarbonyl stehen,
oder
- R² und R³: bilden gemeinsam mit dem Stickstoffatom, an das sie gebunden sind, einen gegebenenfalls durch C₁-C₆-Alkyl oder C₁-C₆-Alkoxy substituierten und gegebenenfalls ein oder zwei weitere Heteroatome aus der Reihe Sauerstoff, Stickstoff und Schwefel enthaltenden 3 bis 7 gliedrigen Ring.

Besonders bevorzugte Substituenten bzw. Bereiche der in den oben erwähnten Verbindungen (Ia), (Ib) und (Ic) aufgeführten Reste werden im Folgenden erläutert.
- R¹: steht für Wasserstoff oder Methyl.
- R²: steht für Wasserstoff, C₁-C₄-Alkyl, C₁-C₄-Halogenalkyl, C₁-C₄-Alkoxy, C₂-C₃-Alkenyl, C₁-C₂-Alkoxy-C₁-C₂-alkyl, C₁-C₂-Alkylcarbonyl, C₁-C₂-Alkoxycarbonyl oder für jeweils gegebenenfalls durch Halogen substituiertes Cyclopropylcarbonyl, Cyclopentylcarbonyl oder Cyclohexylcarbonyl.
- R³: steht für Wasserstoff, C₁-C₄-Alkyl, C₁-C₄-Halogenalkyl, Cyano-C₁-C₄-alkyl, C₂-C₄-Alkenyl, C₂-C₄-Halogenalkenyl, C₂-C₄-Alkinyl, C₂-C₄-Halogenalkinyl, C₁-C₄-Alkoxy, C₁-C₄-Halogenalkoxy, gegebenenfalls durch Halogen substituiertes C₁-C₂-Alkoxy-C₁-C₄-alkyl, gegebenenfalls durch Halogen substituiertes Bis(C₁-C₂-alkoxy)-C₁-C₄-alkyl, gegebenenfalls durch Halogen substituiertes C₁-C₄-Alkylthio-C₁-C₄-alkyl, gegebenenfalls durch Halogen substituiertes C₁-C₄-Alkylcarbonyl-C₁-C₄-alkyl, gegebenenfalls durch Halogen substituiertes C₁-C₄-Alkylsulfinyl-C₁-C₄-alkyl, gegebenenfalls durch Halogen substituiertes C₁-C₄-Alkylsulfonyl-C₁-C₄-alkyl, gegebenenfalls durch Halogen substituiertes C₁-C₄-Alkoxycarbonyl-C₁-C₄-alkyl, für C₂-C₄-Alkinyloxy, für C₂-C₄-Alkinyloxycarbonyl, für gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkyl, für gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkylcarbonyl, für gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkyl-C₁-C₄-alkyl, für gegebenenfalls durch Halogen, Cyano (auch im Alkylteil), Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfinyl, C₁-C₄-Alkylsulfonyl, C₁-C₄-Haloalkylsulfinyl, C₁-C₄-Haloalkylsulfonyl, Amino, C₁-C₄-Alkylamino, Di(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₁-C₄-Alkyl-C₃-C₆-cycloalkyl, C₃-C₆-Cycloalkyl-C₁-C₄-alkyl, C₁-C₄-Alkylcarbonyl, C₁-C₄-Alkoxycarbonyl oder Aminocarbonyl substituiertes Heterocyclyl-C₁-C₄-alkyl, für gegebenenfalls durch Halogen, Cyano, C₁-C₄-Alkyl, C₁-C₄-Halogenalkyl, C₁-C₄-Alkoxy oder C₁-C₄-Halogenalkoxy substituiertes Aryl-C₁-C₄-alkyl, für gegebenenfalls durch Halogen, Cyano (auch im Alkylteil), Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfinyl, C₁-C₄-Alkylsulfonyl, C₁-C₄-Haloalkylsulfinyl, C₁-C₄-Haloalkylsulfonyl, Amino, C₁-C₄-Alkylamino, Di(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₁-C₄-Alkyl-C₃-C₆-cycloalkyl, C₃-C₆-Cycloalkyl-C₁-C₄-alkyl, C₁-C₄-Alkylcarbonyl, C₁-C₄-Alkoxycarbonyl oder Aminocarbonyl substituiertes Hetaryl-C₁-C₄-alkyl
oder
- R² und R³: bilden gemeinsam mit dem Stickstoffatom, an das sie gebunden sind, einen gegebenen-falls durch C₁-C₄-Alkyl oder C₁-C₄-Alkoxy substituierten und gegebenenfalls ein oder zwei weitere Heteroatome aus der Reihe Sauerstoff, Stickstoff und Schwefel enthaltenden 3 bis 7 gliedrigen Ring.

Ganz besonders bevorzugte Substituenten bzw. Bereiche der in den oben erwähnten Verbindungen (Ia), (Ib) und (Ic) aufgeführten Reste werden im Folgenden erläutert.
- R¹: steht für Wasserstoff oder Methyl.
- R²: steht für Wasserstoff, Methyl, Ethyl, CH₂CF₃, CH₂CF₂CH₃, CH₂CHF₂, CH₂CF₂CHF₂, CH₂CH₂F, CH₂-CHF-CH₃, CH₂CF₂Br, CH₂CFCl₂, CH(CH₃)CH₂F, CH₂CCl₃, CH₂CClF₂, CH₂CH₂CH₂F, CH₂CH(CH₃)Cl, CHCF₃CH(CH₃)₂, CH(CF₃)₂, CH₂CH₂Cl, CHCF₃CH₂CH₂CH₃ oder CH₂CF₂CF₃, Methoxy, Ethoxy, Vinyl, CH₂OCH₃, CH₂OCH₂CH₃, Methylcarbonyl, Ethylcarbonyl, Methoxycarbonyl, Ethoxycarbonyl, Cyclopropylcarbonyl oder Fluorcyclpropylcarbonyl.
- R³: steht für Wasserstoff, Methyl, Ethyl, CH₂CF₃, CH₂CF₂CH₃, CH₂CHF₂, CH₂CF₂CHF₂, CH₂CH₂F, CH₂-CHF-CH₃, CH₂CF₂Br, CH₂CFCl₂, CH(CH₃)CH₂F, CH₂CCl₃, CH₂CClF₂, CH₂CH₂CH₂F, CH₂CH(CH₃)Cl, CHCF₃CH(CH₃)₂, CH(CF₃)₂, CH₂CH₂Cl, CHCF₃CH₂CH₂CH₃, CH₂CF₂CF₃, C(CH₃)₂CN, C(CN)CH(CH₃)₂, CH₂CN, CH₂CH₂CN, Vinyl, C(CH₃)₂CCH, CH₂CCCH₃, Methoxy, Ethoxy, CH₂CH(CH₃)(OCH₃), CH₂C(CH₃)₂(OCH₃), CH(CH₃)CH(OCH₃)₂, CH₂C(CH₃)(OCH₃)₂, C(CH₃)₂-CH₂SCH₃, CH₂CH₂SCH₃, CHCH₃CH₂SCH₃, gegebenenfalls durch Halogen substituiertes C₁-C₄-Alkylcarbonyl-C₁-C₄-alkyl, CH₃SO₂CH₂C(CH₃)₂, CH₃SO₂CH₂CHCH₃, Propargyloxy, Cyanocyclopropyl, Fluorcyclopropyl, Trifluormethylcyclopropyl, Trifluormethylcyclohexyl, Methoxycarbonylcyclopropyl, Fluorcyclopropylcarbonyl, Cyclopropylmethyl, Cyclohexylmethyl, 1-Cyano-1-cyclopropyl-eth-1-yl, 1,3-Dioxolan-2-ylmethyl, 4-Methyl-1,3-dioxolan-2-ylmethyl, Tetrahydrofurylmethyl, Tetrahydropyranylmethyl, 2,2-Dimethyl-1,3-dioxolan-5-yl-methyl, 2-Methyltetrahydrofur-2-yl-methyl, α-Methyl-3,5-dimethyltriazol-1-yl-ethyl, 1,5-Dimethyl-1,3-oxazol-4-yl-methyl, für gegebenenfalls durch Halogen, Cyano, Methyl, Ethyl, Methoxy oder Ethoxy substituiertes Benzyl oder für gegebenenfall durch Halogen, Cyano, Methyl, Methoxy oder Ethoxy substituiertes Pyrimidylmethyl, (insbesondere Pyrimid-2-yl-methyl, α-Methyl-pyrimidylmethyl, 4-Brom-pyrimid-2-yl-methyl, 2-Methylpyrimid-4-yl-methyl, 4,6-Dimethylpyrimid-2-yl-methyl, 4-lod-pyrimid-2-yl-methyl, 2-Ethyl-pyrimid-6-yl-methyl, 5-Chlor-pyrimid-2-yl-methyl, 5-Brom-pyrimid-2-yl-methyl, 5-Cyano-pyrimid-2-yl-methyl, 4,6-Dimethoxy-pyrimid-2-yl-methyl und 4,6-Diethoxy-2-pyrimid-2-yl-methyl), Oxadiazolylmethyl, Oxazolylmethyl, 5-Methyl-pyrazin-2-yl, α-Methyl-pyrid-2-yl-methyl, Imidazolylmethyl, 6-Chlor-pyridin-3-yl-methyl, Thiazolylmethyl, Furanylmethyl, 1,5-Dimethylpyrazol-3-yl-methyl, 3-Cyclopropyl-1,2,4-oxadiazol-5-yl-methyl, 6-Brom-pyrid-2-yl-methyl,
oder
- R² und R³: bilden gemeinsam mit dem Stickstoffatom, an das sie gebunden sind, einen gegebenenfalls durch C₁-C₄-Alkyl oder C₁-C₄-Alkoxy substituierten und gegebenenfalls ein oder zwei weitere Heteroatome aus der Reihe Sauerstoff, Stickstoff und Schwefel enthaltenden 3 bis 7 gliedrigen Ring, beispielsweise stehen R² und R³ gemeinsam für CH₂CH₂CH₂ oder CH₂CH₂CH₂O.

Bevorzugte Substituenten bzw. Bereiche der in den oben erwähnten Verbindungen (Id) aufgeführten Reste werden im Folgenden erläutert.
- R¹: steht für Wasserstoff oder C₁-C₆-Alkyl und hier wiederum bevorzugt für Wasserstoff oder Methyl.
- R²: steht für Wasserstoff, C₁-C₆-Alkyl, C₁-C₆-Halogenalkyl, C₁-C₄-Alkoxy, C₁-C₄-Halogenalkoxy, C₂-C₄-Alkenyl, C₁-C₄-Alkoxy-C₁-C₄-alkyl, gegebenenfalls durch Halogen substituiertes C₁-C₄-Alkylcarbonyl, gegebenenfalls durch Halogen substituiertes C₁-C₄-Alkoxycarbonyl oder gegebenenfalls durch Halogen substituiertes C₃-C₆-Cycloalkylcarbonyl.
- R³: steht für C₁-C₆-Halogenalkyl, Cyano-C₁-C₆-alkyl, C₂-C₄-Halogenalkenyl, C₂-C₄-Halogenalkinyl, C₁-C₆-Alkoxy, C₁-C₆-Halogenalkoxy, gegebenenfalls durch Halogen substituiertes C₁-C₆-Alkoxy-C₁-C₆-alkyl, gegebenenfalls durch Halogen substituiertes Bis(C₁-C₆-alkoxy)-C₁-C₆-alkyl, gegebenenfalls durch Halogen substituiertes C₁-C₆-Alkylthio-C₁-C₆-alkyl, gegebenenfalls durch Halogen substituiertes C₁-C₄-Alkylcarbonyl-C₁-C₄-alkyl, gegebenenfalls durch Halogen substituiertes C₁-C₆-Alkylsulfmyl-C₁-C₆-alkyl, gegebenenfalls durch Halogen substituiertes C₁-C₆-Alkylsulfonyl-C₁-C₆-alkyl, gegebenenfalls durch Halogen substituiertes C₁-C₆-Alkoxycarbonyl-C₁-C₆-alkyl, für C₂-C₄-Alkinyloxy, für C₂-C₄-Alkinyloxycarbonyl, für durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl, für gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkylcarbonyl, für gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl-C₁-C₆-alkyl, für gegebenenfalls durch Halogen, Cyano (auch im Alkylteil), Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Alkylsulfmyl, C₁-C₆-Alkylsulfonyl, C₁-C₆-Haloalkylsulfinyl, C₁-C₆-Haloalkylsulfonyl, Amino, C₁-C₆-Alkylamino, Di(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkyl-C₃-C₆-cycloalkyl, C₁-C₆-Alkylcarbonyl, C₁-C₆-Alkoxycarbonyl oder Aminocarbonyl substituiertes Heterocyclyl-C₁-C₆-alkyl, für durch Halogen, Cyano, C₁-C₆-Alkyl, C₁-C₆-Halogenalkyl, C₁-C₆-Alkoxy oder C₁-C₆-Halogenalkoxy substituiertes Aryl-C₁-C₆-alkyl, oder für gegebenenfalls durch Halogen, Cyano (auch im Alkylteil), Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Alkylsulfmyl, C₁-C₆-Alkylsulfonyl, C₁-C₆-Haloalkylsulfinyl, C₁-C₆-Haloalkylsulfonyl, Amino, C₁-C₆-Alkylamino, Di(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkyl-C₃-C₆-cycloalkyl, C₁-C₆-Alkylcarbonyl, C₁-C₆-Alkoxycarbonyl oder Aminocarbonyl substituiertes Hetaryl-C₁-C₆-alkyl.

Besonders bevorzugte Substituenten bzw. Bereiche der in den oben erwähnten Verbindungen (Id) aufgeführten Reste werden im Folgenden erläutert.
- R¹: steht für Wasserstoff oder Methyl.
- R²: steht für Wasserstoff, C₁-C₄-Alkyl, C₁-C₄-Halogenalkyl, C₁-C₄-Alkoxy, C₂-C₃-Alkenyl, C₁-C₂-Alkoxy-C₁-C₂-alkyl, C₁-C₂-Alkylcarbonyl, C₁-C₂-Alkoxycarbonyl oder für jeweils gegebenenfalls durch Halogen substituiertes Cyclopropylcarbonyl, Cyclopentylcarbonyl oder Cyclohexylcarbonyl.
- R³: steht für C₁-C₄-Halogenalkyl, Cyano-C₁-C₄-alkyl, C₂-C₄-Halogenalkenyl, C₂-C₄-Halogenalkinyl, C₁-C₄-Alkoxy, C₁-C₄-Halogenalkoxy, gegebenenfalls durch Halogen substituiertes C₁-C₂-Alkoxy-C₁-C₄-alkyl, gegebenenfalls durch Halogen substituiertes Bis(C₁-C₂-alkoxy)-C₁-C₄-alkyl, gegebenenfalls durch Halogen substituiertes C₁-C₄-Alkylthio-C₁-C₄-alkyl, gegebenenfalls durch Halogen substituiertes C₁-C₄-Alkylcarbonyl-C₁-C₄-alkyl, gegebenenfalls durch Halogen substituiertes C₁-C₄-Alkylsulfinyl-C₁-C₄-alkyl, gegebenenfalls durch Halogen substituiertes C₁-C₄-Alkylsulfonyl-C₁-C₄-alkyl, gegebenenfalls durch Halogen substituiertes C₁-C₄-Alkoxycarbonyl-C₁-C₄-alkyl, für C₂-C₄-Alkinyloxy, für C₂-C₄-Alkinyloxycarbonyl, für durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkyl, für gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkylcarbonyl, für gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkyl-C₁-C₄-alkyl, für gegebenenfalls durch Halogen, Cyano (auch im Alkyl-teil), Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfinyl, C₁-C₄-Alkylsulfonyl, C₁-C₄-Haloalkylsulfinyl, C₁-C₄-Haloalkylsulfonyl, Amino, C₁-C₄-Alkylamino, Di(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkyl-C₃-C₆-cycloalkyl, C₁-C₄-Alkylcarbonyl, C₁-C₄-Alkoxycarbonyl oder Aminocarbonyl substituiertes Heterocyclyl-C₁-C₄-alkyl, für durch Halogen, Cyano, C₁-C₄-Alkyl, C₁-C₄-Halogenalkyl, C₁-C₄-Alkoxy oder C₁-C₄-Halogenalkoxy substituiertes Aryl-C₁-C₄-alkyl oder für gegebenenfalls durch Halogen, Cyano (auch im Alkylteil), Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfinyl, C₁-C₄-Alkylsulfonyl, C₁-C₄-Haloalkylsulfinyl, C₁-C₄-Haloalkylsulfonyl, Amino, C₁-C₄-Alkylamino, Di(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkyl-C₃-C₆-cycloalkyl, C₁-C₄-Alkylcarbonyl, C₁-C₄-Alkoxycarbonyl oder Amino-carbonyl substituiertes Hetaryl-C₁-C₄-alkyl.

Ganz besonders bevorzugte Substituenten bzw. Bereiche der in den oben erwähnten Verbindungen (Id) aufgeführten Reste werden im Folgenden erläutert.
- R¹: steht für Wasserstoff oder Methyl.
- R²: steht für Wasserstoff, Methyl, Ethyl, CH₂CF₃, CH₂CF₂CH₃, CH₂CHF₂, CH₂CF₂CHF₂, CH₂CH₂F, CH₂-CHF-CH₃, CH₂CF₂Br, CH₂CFCl₂, CH(CH₃)CH₂F, CH₂CCl₃, CH₂CClF₂, CH₂CH₂CH₂F, CH₂CH(CH₃)Cl, CHCF₃CH(CH₃)₂, CH(CF₃)₂, CH₂CH₂Cl, CHCF₃CH₂CH₂CH₃ oder CH₂CF₂CF₃, Methoxy, Ethoxy, Vinyl, CH₂OCH₃, CH₂OCH₂CH₃, Methylcarbonyl, Ethylcarbonyl, Methoxycarbonyl, Ethoxycarbonyl, Cyclopropylcarbonyl oder Fluorcyclopropylcarbonyl.
- R³: steht für CH₂CF₃, CH₂CF₂CH₃, CH₂CHF₂, CH₂CF₂CHF₂, CH₂CH₂F, CH₂-CHF-CH₃, CH₂CF₂Br, CH₂CFCl₂, CH(CH₃)CH₂F, CH₂CCl₃, CH₂CClF₂, CH₂CH₂CH₂F, CH₂CH(CH₃)Cl, CHCF₃CH(CH₃)₂, CH(CF₃)₂, CH₂CH₂Cl, CHCF₃CH₂CH₂CH₃, CH₂CF₂CF₃, C(CH₃)₂CN, C(CN)CH(CH₃)₂, CH₂CN, CH₂CH₂CN, Methoxy, Ethoxy, CH₂CH(CH₃)(OCH₃), CH₂C(CH₃)₂(OCH₃), CH(CH₃)CH(OCH₃)₂, CH₂C(CH₃)(OCH₃)₂, C(CH₃)₂-CH₂SCH₃, CH₂CH₂SCH₃, CHCH₃CH₂SCH₃, gegebenenfalls durch Halogen substituiertes C₁-C₄-Alkylcarbonyl-C₁-C₄-alkyl, CH₃SO₂CH₂C(CH₃)₂, CH₃SO₂CH₂CHCH₃, Propargyloxy, Cyanocyclopropyl, Fluorcyclopropyl, Trifluormethylcyclopropyl, Trifluormethylcyclohexyl, Methoxycarbonylcyclopropyl, Fluorcyclopropylcarbonyl, Cyclopropylmethyl, Cyclohexylmethyl, 1-Cyano-1-cyclopropyl-eth-1-yl, 1,3-Dioxolan-2-ylmethyl, 4-Methyl-1,3-Dioxolan-2-ylmethyl, Tetrahydrofurylmethyl, Tetrahydropyranylmethyl, 2,2-Dimethyl-1,3-dioxolan-5-yl-methyl, 2-Methyltetrahydrofur-2-yl-methyl, α-Methyl-3,5-dimethyltriazol-1-yl-ethyl, 1,5-Dimethyl-1,3-oxazol-4-yl-methyl, für gegebenenfalls durch Halogen, Cyano, Methyl, Ethyl, Methoxy oder Ethoxy substituiertes Benzyl, oder für gegebenenfall durch Halogen, Cyano, Methyl, Methoxy oder Ethoxy substituiertes Pyrimidylmethyl, (insbesondere Pyrimid-2-yl-methyl, α-Methyl-pyrimidylmethyl, 4-Brom-pyrimid-2-yl-methyl, 2-Methylpyrimid-4-yl-methyl, 4,6-Dimethylpyrimid-2-yl-methyl, 4-lod-pyrimid-2-yl-methyl, 2-Ethyl-pyrimid-6-yl-methyl, 5-Chlor-pyrimid-2-yl-methyl, 5-Brom-pyrimid-2-yl-methyl, 5-Cyano-pyrimid-2-yl-methyl, 4,6-Dimethoxy-pyrimid-2-yl-methyl und 4,6-Diethoxy-2-pyrimid-2-yl-methyl), Oxadiazolylmethyl, Oxazolylmethyl, 5-Methyl-pyrazin-2-yl, α-Methyl-pyrid-2-yl-methyl, Imidazolylmethyl, 6-Chlor-pyridin-3-yl-methyl, Thiazolylmethyl, Furanylmethyl, 1,5-Dimethylpyrazol-3-yl-methyl, 3-Cyclopropyl-1,2,4-oxadiazol-5-yl-methyl, 6-Brom-pyrid-2-yl-methyl.

Bevorzugte Substituenten bzw. Bereiche der in den oben erwähnten Verbindungen (Ie) aufgeführten Reste werden im Folgenden erläutert.
- R²: steht für Wasserstoff oder C₁-C₆-Alkyl.
- R³: steht für Wasserstoff oder C₁-C₆-Alkyl.

Besonders bevorzugte Substituenten bzw. Bereiche der in den oben erwähnten Verbindungen (Ie) aufgeführten Reste werden im Folgenden erläutert.
- R²: steht für Wasserstoff oder C₁-C₄-Alkyl.
- R³: steht für Wasserstoff oder C₁-C₄-Alkyl.

Ganz besonders bevorzugte Substituenten bzw. Bereiche der in den oben erwähnten Verbindungen (Ie) aufgeführten Reste werden im Folgenden erläutert.
- R²: steht für Wasserstoff, Methyl, Ethyl, n-Propyl oder i-Propyl.
- R³: steht für Wasserstoff, Methyl, Ethyl, n-Propyl oder i-Propyl.

In den bevorzugten Definitionen ist, sofern nichts anderes angegeben ist,

Halogen ausgewählt aus der Reihe Fluor, Chlor, Brom und Iod, bevorzugt wiederum aus der Reihe Fluor, Chlor und Brom,

Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,

Hetaryl (auch als Teil einer größeren Einheit, wie beispielsweise Hetarylalkyl) ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl,

Heterocyclyl (auch als Teil einer größeren Einheit, wie beispielsweise Heterocyclylalkyl) ausgewählt aus der Reihe 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5 Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,3-Dihydrothien-2-yl, 2,3-Dihydrothien-3-yl, 2,4-Dihydrothien-2-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl.

In den besonders bevorzugten Definitionen ist, sofern nichts anderes angegeben ist,

Halogen ausgewählt aus der Reihe Fluor, Chlor, Brom und Iod, bevorzugt wiederum aus der Reihe Fluor, Chlor und Brom,

Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,

Hetaryl (auch als Teil einer größeren Einheit, wie beispielsweise Hetarylalkyl) ausgewählt aus der Reihe Pyrimidyl, Oxadiazolyl, Oxazolyl, Pyrazinyl, Imidazolyl, Thiazolyl und Furanyl,

Heterocyclyl (auch als Teil einer größeren Einheit, wie beispielsweise Heterocyclylalkyl) ausgewählt aus der Reihe 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5 Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,3-Dihydrothien-2-yl, 2,3-Dihydrothien-3-yl, 2,4-Dihydrothien-2-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl.

Durch Halogen substituierte Reste, z.B. Haloalkyl (= Halogenalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Bevorzugt, besonders bevorzugt oder ganz besonders bevorzugt sind Verbindungen, welche jeweils die unter bevorzugt, besonders bevorzugt oder ganz besonders bevorzugt genannten Substituenten tragen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die oben aufgeführten allgemeinen oder in Vorzugsbereiche aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt werden Verbindungen der Formel (I), in welchen eine Kombination der vorstehend als bevorzugt aufgeführten Bedeutungen vorliegt.

Erfindungsgemäß besonders bevorzugt werden Verbindungen der Formel (I), in welchen eine Kombination der vorstehend als besonders bevorzugt aufgeführten Bedeutungen vorliegt.

Erfindungsgemäß ganz besonders bevorzugt werden Verbindungen der Formel (I), in welchen eine Kombination der vorstehend als ganz besonders bevorzugt aufgeführten Bedeutungen vorliegt.

Eine hervorgehobene Gruppe von erfindungsgemäßen Verbindungen sind die unter (Ia) definierten Verbindungen.

Eine weitere hervorgehobene Gruppe von erfindungsgemäßen Verbindungen sind die unter (Ib) definierten Verbindungen

Eine weitere hervorgehobene Gruppe von erfindungsgemäßen Verbindungen sind die unter (Ic) definierten Verbindungen

Eine weitere hervorgehobene Gruppe von erfindungsgemäßen Verbindungen sind die unter (Id) definierten Verbindungen

Eine weitere hervorgehobene Gruppe von erfindungsgemäßen Verbindungen sind die unter (Ie) definierten Verbindungen

In einer weiteren hervorgehobenen Gruppe von erfindungsgemäßen Verbindungen steht R³ für Halogenalkyl.

In einer weiteren hervorgehobenen Gruppe von erfindungsgemäßen Verbindungen steht R³ für Heterocyclylalkyl.

In einer weiteren hervorgehobenen Gruppe von erfindungsgemäßen Verbindungen steht R³ für Hetarylalkyl.

In einer weiteren hervorgehobenen Gruppe von erfindungsgemäßen Verbindungen steht X für Sauerstoff.

In einer weiteren hervorgehobenen Gruppe von erfindungsgemäßen Verbindungen steht X für Schwefel.

Die Herstellung erfindungsgemäßer Verbindungen der Formel (I), in welcher G² für den Rest (A) steht, und entsprechender Vorstufen werden in den folgenden Reaktionsschemata erläutert.

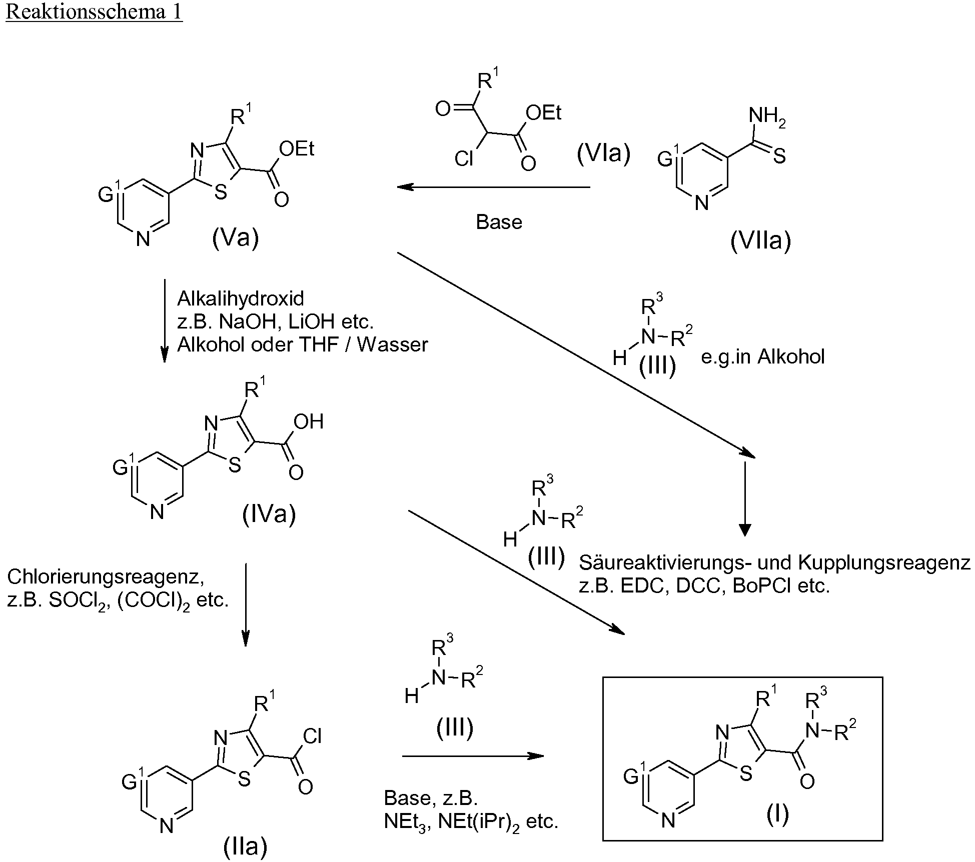

Die als Ausgangsstoffe benötigten Verbindungen der Formel (VIIa), in welcher G¹ die oben genannte Bedeutung hat, sind bekannt bzw. nach bekannten Methoden erhältlich, beispielsweise wie in Synthetic Communications 35, 5, 2005, 761 beschrieben. Verbindungen der Formel (Va) in welchen R¹ für Alkyl steht, sind herstellbar unter Verwendung der in Reaktionsschema 1 angegebenen Standardmethoden, vgl. DE 2221647. Umsetzung eines Thioamids (VIIa) mit dem Ester der Formel (VIa) in Gegenwart einer Base wie beispielsweise Triethylamin liefert das Thiazol (Va). Die Verbindungen der Formel (Va) in welchen R¹ für Wasserstoff steht, sind herstellbar in Analogie zu den Methoden, die in Helvetica Chimica Acta 1944, 1432-1436 beschrieben sind. Der dort eingesetzte Chlorformylester kann hergestellt werden wie in Chemische Berichte, 1910, 3528-3533 beschrieben. Bevorzugt aber wird das in Analogie zu dem dort beschriebenen Kaliumsalz hergestellte Natriumsalz des Chlorformylesters direkt für die Umsetzung mit einem Thioamid der Formel (VIIa) ohne Zusatz einer Base verwendet, siehe Beispiel 3.

Das Thiazol der Formel (Va) kann unter Verwendung der in Reaktionsschema 1 angegebenen Standardmethoden, vgl. DE 2221647, zunächst in die Säure der Formel (IVa) und anschließend in das Säurechlorid der Formel (IIa) überführt werden. Weitere Umsetzung mit dem Amin der Formel (III), in welcher R² und R³ die oben angegebenen Bedeutungen haben, in einem Verdünnungsmittel wie beispielsweise Dichlormethan oder Tetrahydrofuran und in Gegenwart einer Base wie beispielsweise Triethylamin oder Diisopropylethylamin, führt zu erfindungsgemäßen Verbindungen der Formel (I), in welchen X für Sauerstoff steht.

Die Verbindungen der Formel (I) lassen sich auch direkt aus den Säuren der Formel (IVa) durch Umsetzung mit Aminen der Formel (III) in Gegenwart von Kupplungsagenzien wie beispielsweise EDC (1-Ethyl-3-[3-dimethylaminopropyl]carbodiimidehydrochlorid), DCC (Dicyclohexylcarbodiimid) oder BoPCl (Bis(2-oxo-3-oxazolidinyl)phosphisäurechlorid) herstellen.

Die Verbindungen der Formel (I) lassen sich auch direkt aus den Estern der Formel (VIa) durch Umsetzung mit Aminen der Formel (III) in einem Verdünnungsmittel wie beispielsweise Ethanol unter Erwärmen herstellen.

Verbindungen der Formel (I), in welchen R² für Wasserstoff steht oder R³ für Wasserstoff steht, können durch Alkylierung, beispielsweise durch Umsetzung mit einem Alkylierungsmittel wie Methyliodid in einem Verdünnungsmittel wie DMF unter Verwendung einer Base (z.B. Natriumhydrid) derivatisiert werden.

Die Amine der Formel (III) sind bekannt oder lassen sich nach im Prinzip bekannten Methoden herstellen, beispielsweise durch Reduktion von Amiden, wie in EP 0 030 092, Seite 86 beschrieben, vergleiche auch Herstellungsbeispiel 1.

Alternativ können Verbindungen der Formel (I) auch durch Suzuki-Kupplung gemäß Reaktionsschema 2 hergestellt werden. Hierbei wird die Säure der Formel (Xa) (vgl. beispielsweise Helvetica Chim Acta 27, 1432 (1944) und J.Het.Chem. 22(6) 1621 (1985)) entweder direkt oder nach Umsetzung zum Säurechlorid der Formel (XIa) in das Amid der Formel (IXa) überführt und dieses anschließend mit dem Borsäureester der Formel (VIIIa) zu erfindungsgemäßen Verbindungen der Formel (I) gekuppelt (vgl. beispielsweise J.Med.Chem 48 (1), 224 (2005)). Die Borverbindungen der Formel (VIIIa) sind bekannt. bzw. nach bekannten Methoden erhältlich (siehe zum Beispiel Tetrahedron 57 (49), 9813 (2001), Journal of Organic.Chemistry 70 (15), 6034 (2005) sowie Tetrahedron Letters 43, (2002), 4285-4287 und Journal of Organic Chemistry 2002, 67, 5394-5397).

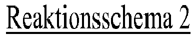

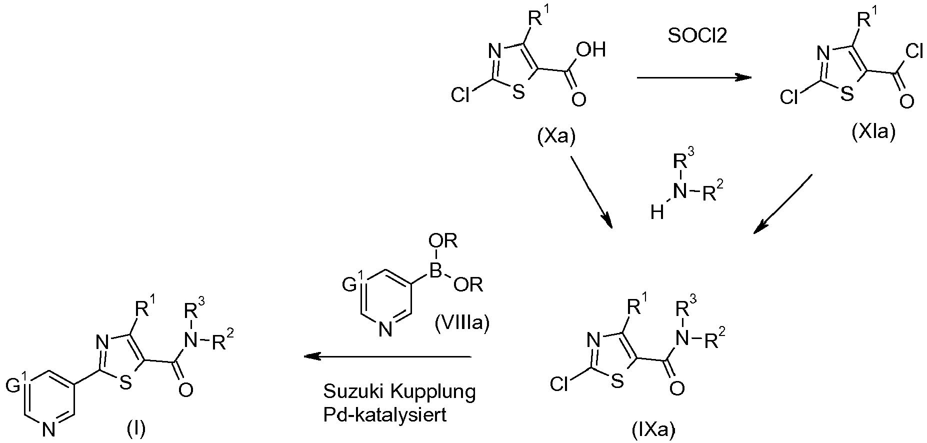

Verbindungen der Formel (I), in denen X für Schwefel steht, lassen sich aus den Verbindungen der Formel (I), in denen X für Sauerstoff steht, durch Umsetzung mit einem Schwefelungsreagenz, beispielsweise Lawessons Reagenz oder P₄S₁₀, herstellen. N-Oxide lassen sich beispielsweise durch Umsetzung von Verbindungen der Formel (I) mit mCPBA (meta-Chlorperbenzoesäure) gewinnen. Salze von Verbindungen der Formel (I) sind gemäß Reaktionsschema 3 (dort beispielhaft für den Rest (A) dargestellt) zugänglich, indem man Verbindungen der Formel (I) mit Verbindungen der Formel RX umsetzt, in der beispielsweise X für Halogen wie Chlor oder Brom und R für einen jeweils gegebenenfalls substituierten Alkyl-, Alkenyl- oder Alkinylrest steht (Reaktionsschema 3).

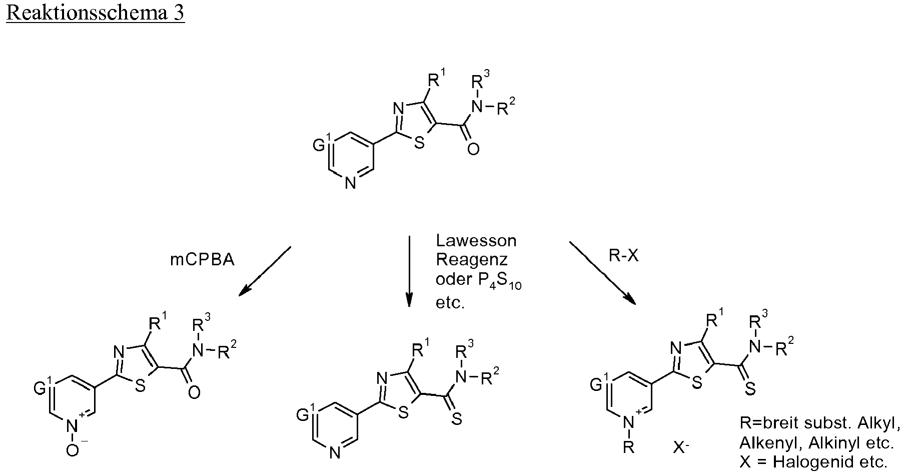

Die Herstellung erfindungsgemäßer Verbindungen der Formel (I), in welcher G² für den Rest (B) steht, und entsprechender Vorstufen werden in den folgenden Reaktionsschemata erläutert.

Die Reaktionsfolge ausgehend vom Ester der Formel (Vb) entspricht der im Reaktionsschema 1, dort ausgehend vom Ester der Formel (Va), und es gelten entsprechend die dort gemachten Angaben (Reaktionsschema 4).

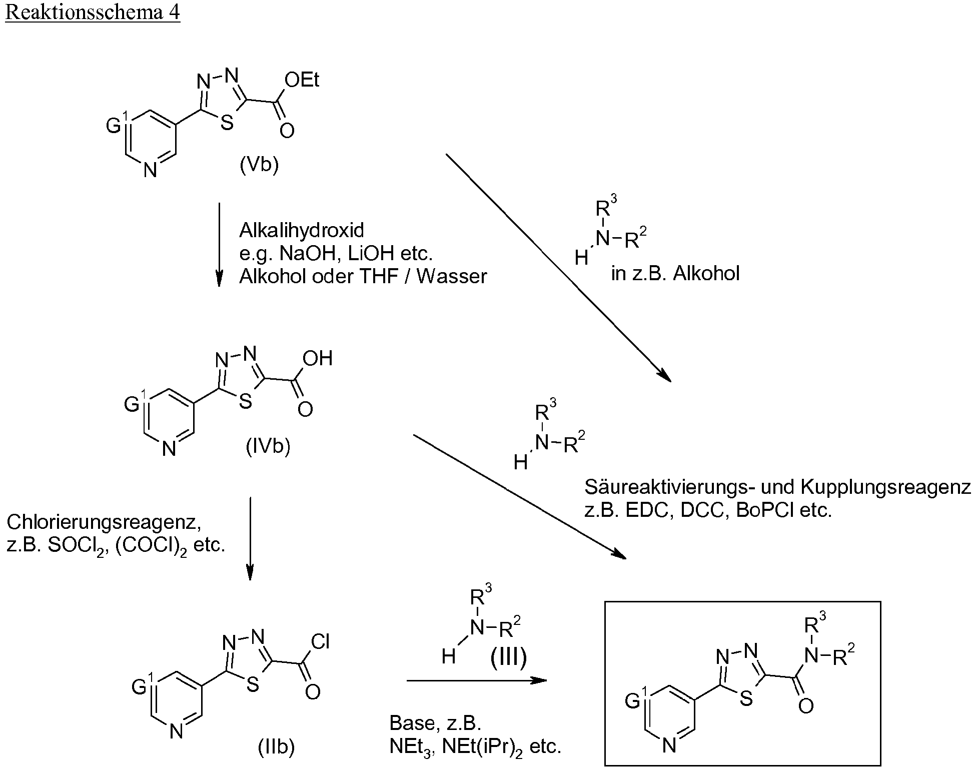

Die Ester der Formel (Vb) sind neu, sie können nach im Prinzip bekannten Verfahren, beispielsweise in Analogie zu Liquid Crystals Today, Vol 14,1, 2005, 15-18 hergestellt werden, beispielsweise wie in Reaktionsschema 5 dargestellt.

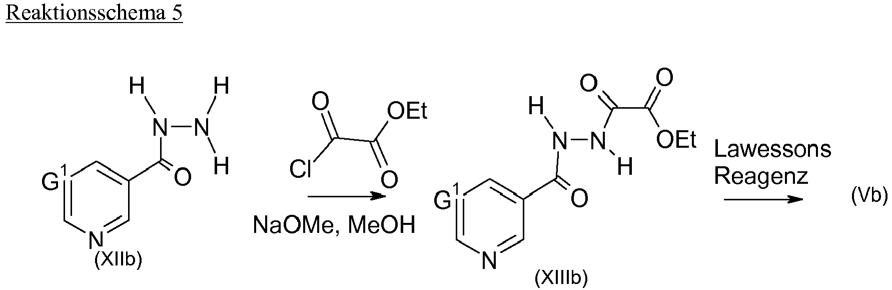

Das Hydrazid der Formel (XIIb) wird in Gegenwart einer Base wie z.B. Triethylamin oder Natriummethanolat in einem Verdünnungsmittel wie beispielsweise Dimethylformamid (DMF) oder Methanol mit Oxalsäurechloridethylester zur Diacylhydrazinverbindung der Formel (XIIIb) umgesetzt, die anschließend in einem Verbindungsmittel wie z.B. mit Lawessons Reagenz zum Thiadiazol der Formel (Vb) reagiert. Hydrazide der Formel (XIIb) sind beschrieben, beispielsweise in Journal of Medicinal Chemistry, 32, 3, 1989, 583-593 und durch Hydrazinolyse der entsprechenden Carbonsäureester erhältlich. Neu und Gegenstand der Erfindung sind auch die Verbindungen der Formeln (IIb), (IVb) und (Vb).

Die Herstellung erfindungsgemäßer Verbindungen der Formel (I), in welcher G² für den Rest (C) steht, und entsprechender Vorstufen werden in den folgenden Reaktionsschemata erläutert.

Die Reaktionsfolge ausgehend vom Ester der Formel (Vc) entspricht der im Reaktionsschema 1, dort ausgehend vom Ester der Formel (Va), und es gelten wiederum entsprechend die dort gemachten Angaben (Reaktionsschema 6).

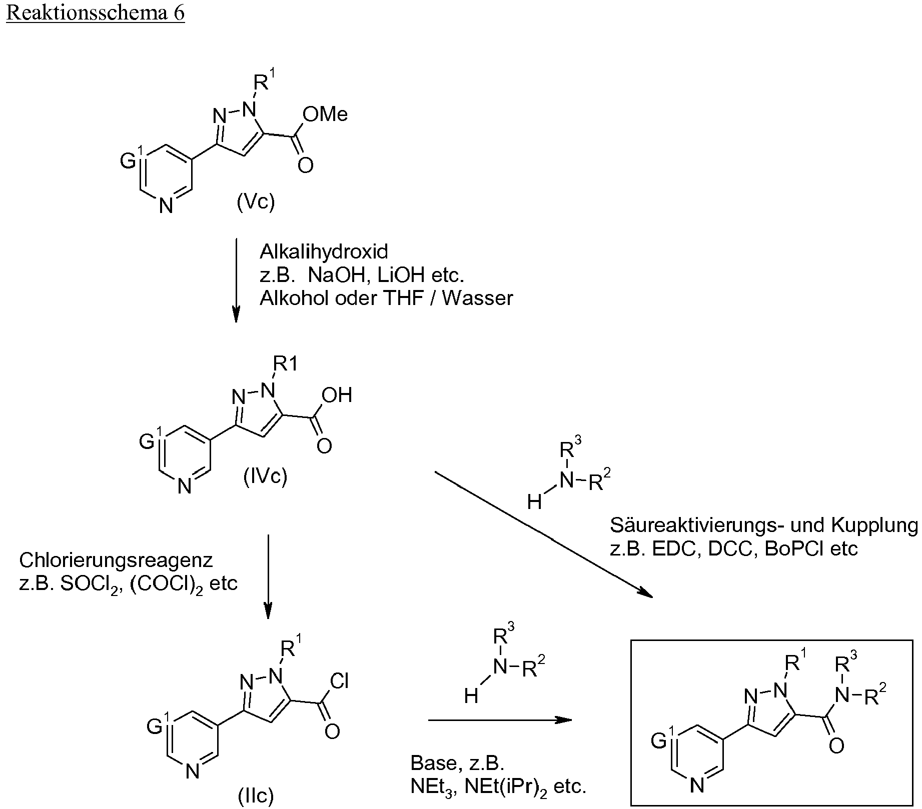

Verbindungen der Formeln (IVc) und (Vc), in welchen R¹ für Wasserstoff steht, sind bekannt (vgl. WO 2005/090328; WO 1999/62885; Bioorganic & Medicinal Chemistry Letters (2000), 10 (11), 1211-1214; J. Chem. Soc. (1933), 350). Verbindungen der Formeln (IVc) und (Vc), in welchen R¹ für Alkyl, insbesondere Methyl, steht, sind neu und auch Gegenstand der Erfindung.

Die Herstellung des Esters der Formel (Vc) ist in Reaktionsschema 7 dargestellt.

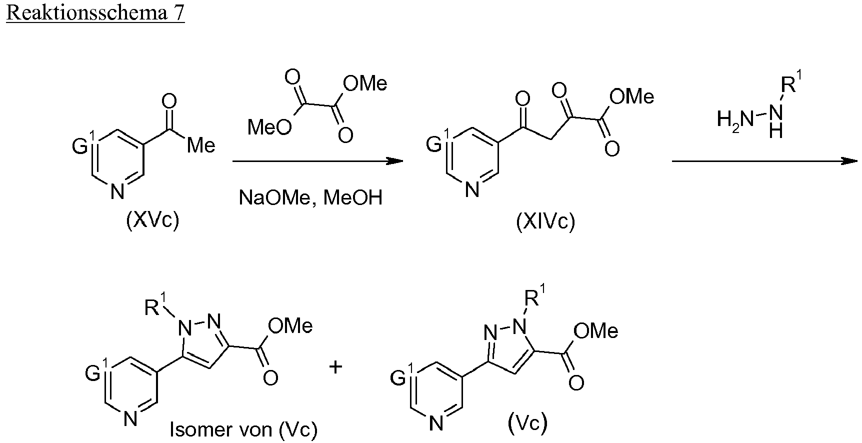

Es wird zunächst der Diketoester der Formel (XVIc) hergestellt, indem das Methylketon der Formel (XVc) mit dem Oxalsäurediester in Gegenwart von Natriummethanolat in Methanol umgesetzt wird (siehe auch WO 2004/002409 und WO 2004/016741). Anschließende Umsetzung mit dem Hydrazinderivat der Formel H₂NNHR¹ in einem Alkohol, z.B. Methanol, als Verdünnungsmittel führt zum Ester der Formel (Vc) und zum oben dargestellten Isomeren von (Vc). Beide Isomere lassen sich beispielsweise chromatographisch trennen. Die Verbindungen der Formel (XVc) sind bekannt bzw. können nach bekannten Verfahren erhalten werden (vgl. z.B. EP334146, EP1270535, Monatshefte für Chemie 126(6/7), 805 (1995), JOC 66(12), 4340 (2001)).

Verbindungen der Formel (I), in welcher X für Sauerstoff steht, lassen sich mit einem Schwefelungsreagenz in Verbindung der Formel (I) überführen, in welcher X für Schwefel steht.

Als Sulfidierungsmittel (Schwefelungsreagenz) werden bevorzugt Phosphorreagenzien wie beispielsweise Diphosphorpentasulfid (P₂S₅), Diphosphorpentasulfid/Pyridin (P₂S₅/Py), Diphosphorpentasulfid/ Triethylamin (P₂S₅/NEt₃), Diphosphorpentasulfid/Natriumhydrogencarbonat (P₂S₅/NaHCO₃ "Scheeren's Reagenz) oder besonders bevorzugt das 2,4-Bis(4-methoxyphenyl)-2,4-dithioxo-1,3,2,4-dithiadiphosphetan "Lawesson's Reagens (LR)", 2,4-Bis(4-phenoxyphenyl)-2,4-dithioxo-1,3,2,4-dithiadiphosphetan "Belleau's Reagens (BR)" bzw. 2,4-Bis(4-phenylthiophenyl)-2,4-dithioxo-1,3,2,4-dithiadiphosphetan, eingesetzt.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-FettalkoholEther, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, AlkylAryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp. (Ctenocephalides canis, Ctenocephalides felis), Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Avicularüdae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele:

### Beispiel 1

### 2-Chloro-2,2-difluoro-ethyl-ammoniumchlorid

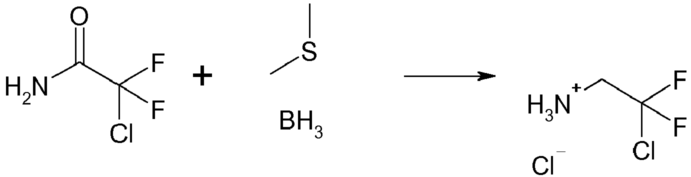

15.12g (116,7 mmol) Chlordifluoracetamid wurden in 150 ml THF unter Argon gelöst und mit 8g (105,3 mmol) Boran-Dimethylsulfid versetzt. Man erhitzte 1 Stunde unter Rückfluß, gab langsam 40 ml verdünnte Salzsäure zu, erhitzte noch 1 Stunde unter Rückfluß, kühlte mit einem Eisbad, versetzte mit Ether, verdünnter Natronlauge und wässriger Zitronensäure bis pH=9, extrahierte die wäßrige Phase insgesamt drei mal mit Ether, trocknete die vereinigten organischen Phasen mit Natriumsulfat, versetzte die erhaltene Lösung mit ca 80 ml HCl 2M in Ether, saugte den gebildeten Niederschlag und trocknete ihn am Rotationsverdampfer.

Ausbeute: 5,99 g (33% d.Th)

### 4-Methyl-2-pyridin-3-yl-thiazol-5-carbonsäure (2-chlor-2,2-difluoro-ethyl)-amid

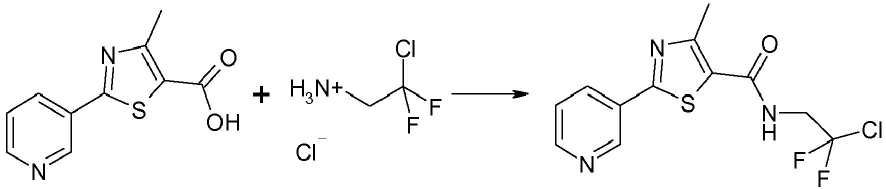

330 mg (1,50 mmol) der Thiazolcarbonsäure und 2,52 g (19,5 mmol) N,N-Diisopropylethylamin wurden in 70 ml Acetonitril vorgelegt und unter Rühren mit 458 mg (1,8 mmol) Bis(2-oxo-3-oxazolidinyl)phosphisäurechlorid (Bop-Cl) versetzt. Nach 20 Minuten wurden 455 mg (3 mmol) des Aminhydrochlorides zugesetzt und 16 Stunden bei Raumtemperatur gerührt. Zur Aufarbeitung wurde im Vakuum eingeengt, zwischen Essigester und Kochsalzlösung/Phosphatpufferlösung (pH 7) verteilt, die organische Phase über Natriumsulfat getrocknet und im Vakuum eingeengt. Der Rückstand wurde chromatographisch an Kieselgel (Cyclohexan / Essigester) gereinigt.

Ausbeute: 265 mg (51 % d.Th.), logP¹⁾ (HCOOH) 1,84 1H-NMR (CD3CN): 2,7(s, 3H) 4,2 (dt, 2H), 7,1 (br, 1H), 7,45 (dd, 1H), 8,2 (m, 1H), 8,75 (m, 1H), 9,1 (m, 1H)

### Beispiel 2

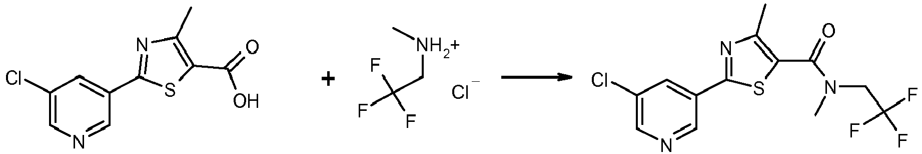

150 mg (0,589 mmol) der Thiazolcarbonsäure und 989 mg (7,66 mmol) N,N-Diisopropylethylamin wurden in 30 ml Acetonitril vorgelegt und unter Rühren mit 179 mg (0,71 mmol) Bis(2-oxo-3-oxazolidinyl)phosphisäurechlorid (Bop-Cl) versetzt. Nach 20 Minuten wurden 264 mg (1,77 mmol) des Aminhydrochlorides zugesetzt und 16 Stunden bei Raumtemperatur gerührt. Zur Aufarbeitung wurde im Vakuum eingeengt, zwischen Essigester und Wasser verteilt, die organische Phase über Magnesiumsulfat getrocknet und im Vakuum eingeengt.

Ausbeute: 192 mg (90 % d.Th.), logP (HCOOH) 2,72

1H-NMR (d6-DMSO): 2,43(s,3H), 3,13(s,3H), 4,35(q,2H), 8,37(1H,m), 8,73(1H,m), 9,05(1H,m) ppm.

### Beispiel 3

### Stufe 1: Natrium 2-chloro-2-ethoxycarbonyl-ethanolat

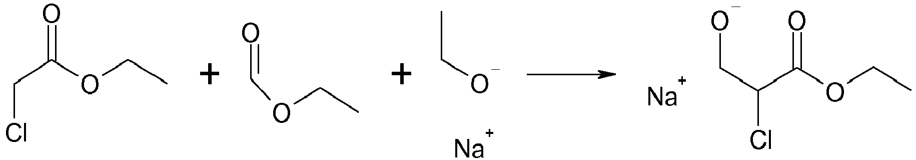

89,31g (728 mmol) Chloressigsäureethylester und 63,7g (859,9 mmol) Ethylformat wurden in 500ml MTBE gelöst. Unter Rühren wurden 259,77g (801 mmol) einer ethanolischen Lösung von Na-Ethanolat zugetropft. Nach 16 Stunden wurde vom gebildeten Niederschlag abdekantiert, der Rückstand mit ca 500 ml Diethylether verrührt, abgesaugt, der Feststoff mit weiteren 500 ml Diethylether gewaschen und getrocknet.

Ausbeute 84 g (66% d. Th.)

### Stufe 2: 2-Pyridin-3-yl-thiazol-5-carbonsäure-ethylester

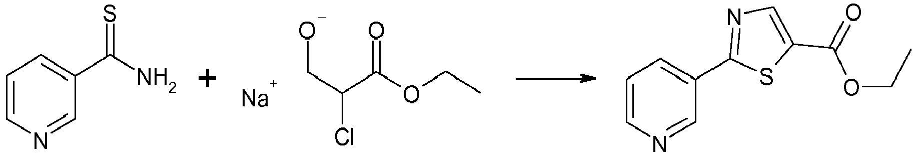

20g (144,72 mmol) Thionikotinamid wurden mit 50,52 g (289,45mmol) Natrium 2-chloro-2-ethoxycarbonyl-ethanolat in 600 ml Ethanol 24 Stunden bei Rückfluß gerührt. Das Lösungsmittel wurde im Vakuum entfernt, der Rückstand mit Dichlormethan versetzt, die organische Phase dreimal mit Wasser gewaschen und eingedampft. Der Rückstand wurde aus Dichlormethan/Hexan umkristallisiert. Aus der Mutterlauge konnte noch weiteres Produkt gewonnen werden.

Ausbeute 18,66 g (50% d.Th.), logP (HCOOH) 1,93

### Stufe 3: 2-Pyridin-3-yl-thiazol-5-carbonsäure

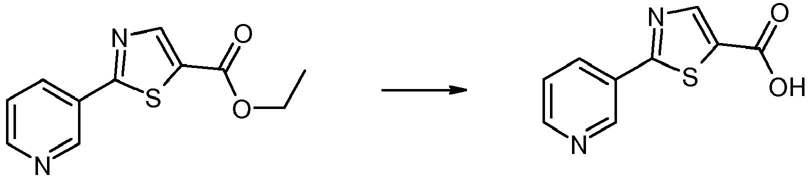

4,03g (16,5 mmol) des Thiazol-carbonsäureesters wurden in 60 ml Ethanol vorgelegt, eine Lösung von 5,86g (66 mmol) Natriumhydroxid in 20 ml Wasser zugetropft und 16 Stunden bei Raumtemperatur gerührt. Das Ethanol wurde abdestilliert und die verbleibende wässrige Lösung mit Salzsäure angesäuert. Das ausgefallene Produkt wurde abgesaugt und getrocknet.

Ausbeute: 2,57 g (75% d.Th.), logP (HCOOH): 0,42

### Stufe 4: 3-(5-Chlorocarbonyl-thiazol-2-yl)-pyridiniumchlorid

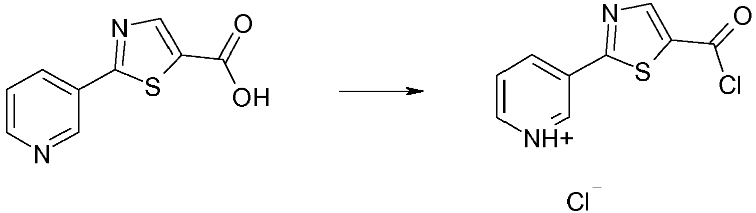

8,50 g (41,21 mmol) der Thiazol-carbonsäure wurden in 120ml Toluol zusammen mit einigen Tropfen DMF mit 5,1 ml (70mmol) Thionylchlorid innerhalb von 30 min versetzt, dann 3 Stunden bei 60°C gerührt, abgekühlt und dabei 30 min Argon durch die Lösung geleitet, noch 30 min in einem Eisbad gekühlt, der Niederschlag abgesaugt, mit Toluol gewaschen und am Rotationsverdampfer getrocknet.

Ausbeute: 10,36 g (96% d.Th.)

### Stufe 5:

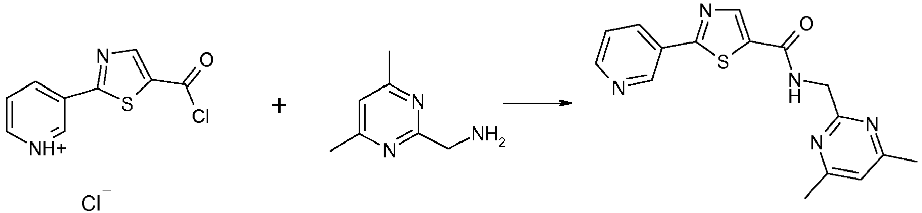

219 mg (0,84 mmol) des Säurechlorids wurden in 6 ml Dioxan vorgelegt, mit 324 mg (2,51 mmol) N,N-Diisopropylethylamin und 138 mg (1,00 mmol) des Pyrimidyl-methylamins versetzt und 16 Stunden bei Raumtemperatur gerührt. Zur Aufarbeitung wurde im Vakuum eingeengt, der Rückstand zwischen Essigester und Wasser verteilt und die organische Phase getrocknet und eingeengt.

Ausbeute: 96 mg (34 % d.Th.), logP (HCOOH) 1,19

1H-NMR(d6-DMSO): 2,40(s,6H), 4,58(m,2H), 7,1(m,1H), 7,55(m,1H), 8,35(m,1H), 8,55(s,1H), 8,70(m,1H), 9,10(m,1H), 9,15(m,1H) ppm.

### Beispiel 4

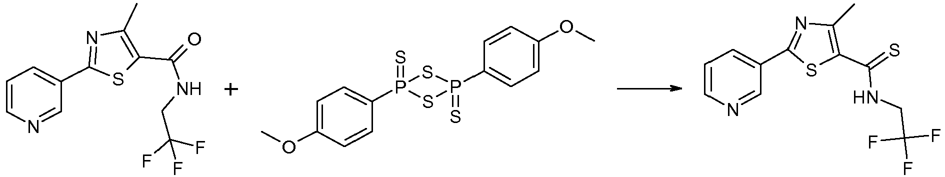

600 mg (1,99 mmol) des Thiazolcarbonsäureamides und 443 mg (1,10 mmol) 4-Methoxyphenyldithiophosphonsäureanhydrid wurden in 60 ml Toluol 16 Stunden unter Argon zum Rückfluß erhitzt. Zur Aufarbeitung wurde zwischen Natriumhydrogencarbonatlösung und Essigester verteilt, die organische Phase getrocknet und eingeengt. Der Rückstand wurde chromatographisch an Kieselgel (Dichlormethan / Essigester) gereinigt.

Ausbeute: 590 mg (92 % d.Th.), logP (HCOOH) 2,39

1H-NMR(d6-DMSO): 2,54(s,3H), 4,65(q,2H), 7,50(m,1H), 8,25(m,1H), 8,65(m,1H), 9,10(m,1H) ppm.

### Beispiel 5

### Stufe 1:

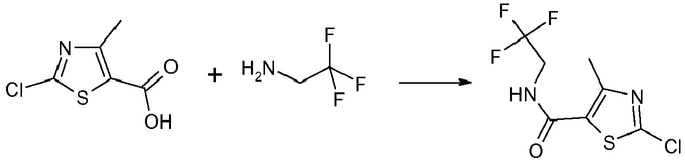

2,22 g ( 12,5 mmol) der Chlorthiazol-carbonsäure und 21 g (162 mmol) Diisopropylethylamin wurden in 50 ml Acetonitril unter Rühren mit 3,82 g (15 mmol) Bis(2-oxo-3-oxazolidinyl)phosphisäurechlorid (Bop-Cl) versetzt. Nach 20 Minuten wurden 1,857 g (18,75 mmol) des Amins zugegeben und 16 Stunden bei Raumtemperatur gerührt. Zur Aufarbeitung wurde zwischen Wasser und Essigester verteilt, die organische Phase getrocknet und eingeengt. Der Rückstand wurde chromatographisch an Kieselgel (Dichlormethan / Essigester) gereinigt.

Ausbeute: 1,10 g (34 % d.Th.), logP (HCOOH) 1,97

### Stufe 2:

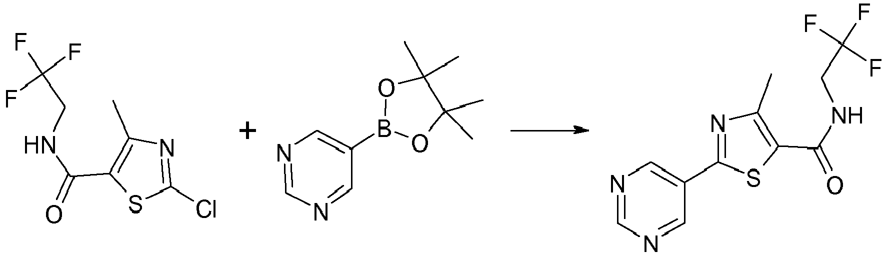

326 mg (1,26 mmol) des Chlorthiazolcarbonsäureamids aus Stufe 1, 200 mg (0,97 mmol) des Pyrimidyl-borsäureesters und 21 mg (0,029 mmol) 1,1'-Bis(diphenylphosphino)ferrocen-palladium(ii)chlorid wurden in 10 ml Dimethoxyethan vorgelegt. Es wurden 1,45 ml 2M Kaliumcarbonatlösung zugesetzt und 4h unter Argon bei 80°C gerührt. Zur Aufarbeitung wurde zwischen Wasser und Dichlormethan verteilt, die organische Phase über Magnesiumsulfat getrocknet und eingeengt. der Rückstand wurde chromatographisch an Kieselgel (Dichlormethan / Essigester) gereinigt.

Ausbeute: 64 mg (21 % d.Th.), logP (HCOOH) 1,61

1H-NMR (d6-DMSO): 2,60(s,3H), 4,05(m,2H), 8,85(m,1H), 9,25(m,2H).

### Beispiel 6

Stufe 1:

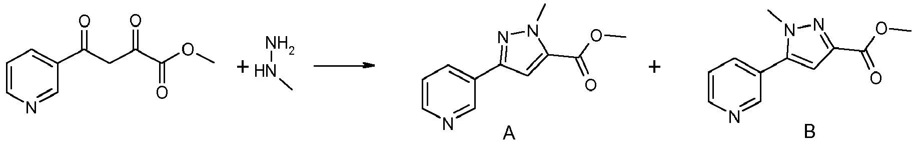

15,0 g (72,4 mmol) des Pyridin-diketoesters und 3,34 g (72,4 mmol) Methylhydrazin wurden 2 Stunden in 300 ml Ethanol unter Rückfluß erhitzt. Dann wurde im Vakuum eingeengt und die Isomeren durch Chromatographie an Kieselgel getrennt (Laufmittel Cyclohexan / Essigester).

### Ausbeute:

6,55 g (41 % d,Th.) des gewünschten Isomers A, logP (HCOOH) 0,84 (weiter eingesetzt in der nächsten Stufe)

2,23 g (14 % d.Th.) des anderen Isomers B, logP (HCOOH) 0,77

### Stufe 2:

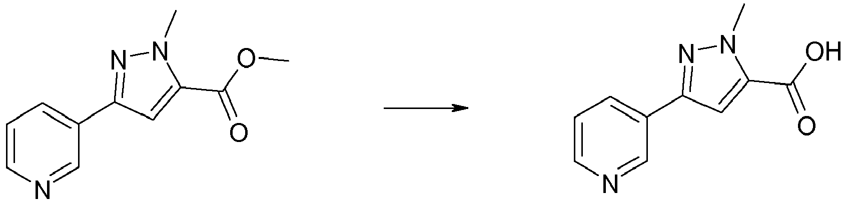

6,55 g (30,15 mmol) des Pyrazolcarbonsäureesters wurden in einem Gemisch aus 400 ml THF (Tetrahydrofuran) und 250 ml Wasser gelöst, mit einer Lösung von 2,53 g (60,31 mmol) Lithiumhydroxid-monohydrat in 150 ml Wasser versetzt und 16 Stunden bei Raumtemperatur gerührt. Zur Aufarbeitung wird das THF im Vakuum entfernt und das ausgefallene Produkt abgesaugt und getrocknet.

Ausbeute: 6,08 g (99% d.Th.)

### Stufe 3:

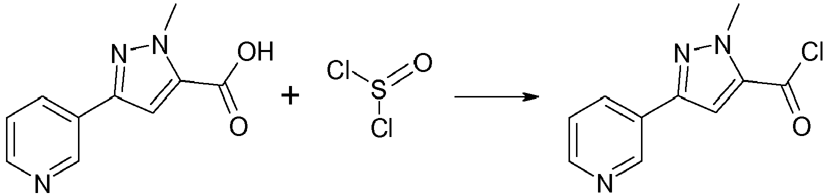

2,0 g (9,84 mmol) der Pyrazolcarbonsäure wurden in 50 ml Thionylchorid 2 Stunden unter Rückfluß erhitzt und eingeengt.

Ausbeute: 2,13 g (91 % d.Th.)

### Stufe 4:

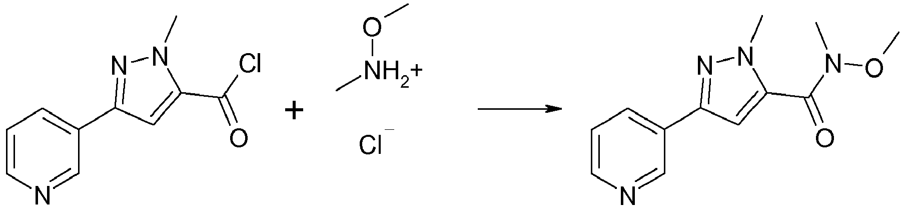

3,00 g (13,5 mmol) des Pyrazolyl-säurechlorids wurden in 100 ml Dioxan mit 7,00 g (54,1 mmol) N,N-Diisopropylethylamin und 1,58 g (16,2 mmol) des Aminhydrochlorids 16 Stunden bei Raumtemperatur gerührt. Zur Aufarbeitung wurde eingeengt und der Rückstand durch Chromatographie an Kieselgel gereinigt (Laufmittel Cyclohexan/Essigester).

Ausbeute: 1,92 g (56 % d.Th.), logP (HCOOH) 0,49

1H-NMR (d6-DMSO): 3,31(s,3H), 3,70(s,3H), 4,02(s,3H), 7,20(s,1H), 7,40(m,1H), 8,15(m,1H), 8,50(m,1H), 9,02(m,1H).

### Beispiel 7

### Stufe 1: Oxo-[N'-(pyridine-3-carbonyl)-hydrazino]-essigsäureethylester:

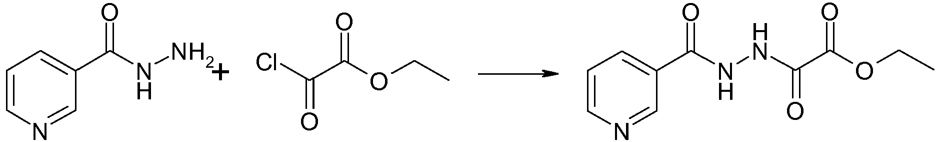

28,0 g (204 mmol) Nikotinsäurehydrazid wurden in 700 ml DMF durch leichtes Erwärmen gelöst, dann 52,5 g (518 mmol) Triethylamin zugesetzt und unter Eisbadkühlung eine Lösung von 31,7 g (232 mmol) Oxalylchlorid in 40 ml Dichlormethan zugetropft. Es wurde 15 Minuten gerührt, eingedampft und der Rückstand zwischen Wasser (pH7) und Chloroform/Isopropanol (10:1) verteilt. Die organische Phase wurde getrocknet und im Vakuum eingeengt.

Ausbeute: 59,0 g Rohprodukt, ohne weitere Reinigung in der nächsten Stufe umgesetzt.

### Stufe 2: 5-Pyridin-3-yl-[1,3,4]thiadiazol-2-carbonsäureethylester

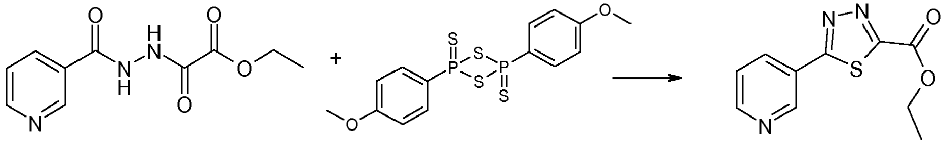

59,0 g (248 mmol) des Hydrazons und 57,3 g (141 mmol) Methoxyphenyldithiophosphonsäureanhydrid wurden in 500 ml Toluol 4 Stunden lang zum Rückfluß erhitzt. Zur Aufarbeitung wurde im Vakuum eingeengt und mittels Chromatographie an Kieselgel (Cyclohexan/Aceton) gereinigt.

Ausbeute: 15,5 g (26% d.Th.), logP (HCOOH) 1,41

### Stufe 3: Natrium- 5-pyridin-3-yl-[1,3,4]thiadiazol-2-carboxylat

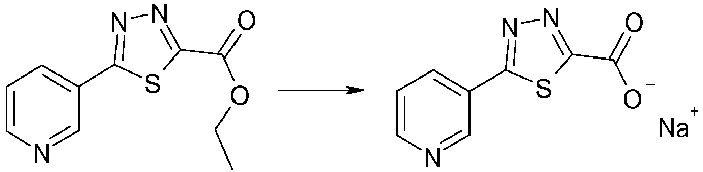

1,5 g (6,37 mmol) des Thiadiazolcarbonsäureesters und 2,3 ml (6,3 mmol) einer 21%igen Lösung von Natriumethylat in Ethanol wurden in einer Mischung aus 23ml Ethanol und 7,5 ml Wasser 30 Minuten bei Raumtemperatur gerührt und noch 10 min bei Eisbadkühlung gerührt. Das ausgefallene Produkt wurde abgesaugt, zweimal mit Ether gewaschen und am Rotationsverdampfer getrocknet.

Ausbeute: 1,35 g (92% d.Th.)

### Stufe 4: 5-Pyridin-3-yl-[1,3,4]thiadiazol-2-carbonsäuredimethylamid

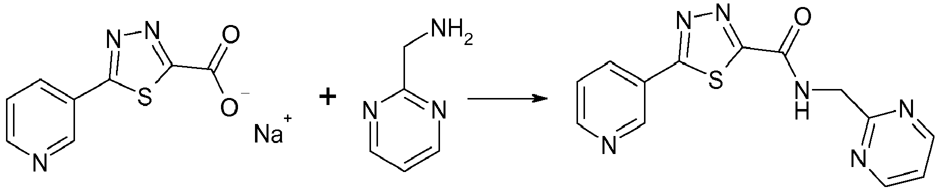

Zu 0,50 g (2,18 mmol) des Thiadiazolcarbonsäuresalzes in einer Mischung aus 20 ml THF und 2 ml DMF (Dimethylformamid) wurden 0,498 g (3,92 mmol) Oxalylchlorid getropft. Nach Beendigung der Gasentwicklung wurden 0,726 g (7,17 mmol) Triethylamin und 0,476 g (4,36 mmol) des Amins zugesetzt und 16 Stunden bei Raumtemperatur gerührt. Zur Aufarbeitung wurde im Vakuum eingeengt, zwischen Wasser und Chloroform/Isopropanol (10:1) verteilt, die organische Phase getrocknet, im Vakuum eingeengt und mittels Chromatographie an Kieselgel (Essigester, Aceton) gereinigt.

Ausbeute: 0,14 g (19 % d.Th.), logP (HCOOH) 0,85; logP (pH=7.5) 0,85

1H-NMR (d6-DMSO): 4,25(m, 2H), 7,4 (t, 1H), 7,6 (m, 1H), 8,4 (m, 1H), 8,8 (m, 3H), 9,2 (m, 1H), 9,4 (br, 1H)

### Beispiel 8

### 5-Pyridin-3-yl-[1,3,4]thiadiazol-2-carbonsäuredimethylamid

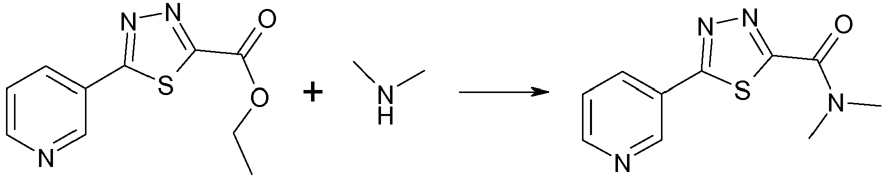

0,3g (1,25 mmol) des Thiadiazolesters wurden mit 2,5 ml (14 mmol) einer 5.6-molaren Lösung von Dimethylamin in Ethanol in 15 ml Ethanol 1 Stunde bei 105°C in einem geschlossenen Reaktionsgefäß gerührt. Man dampfte ein, löste in 10 ml heißem 1,1,1-Benzotrifluorid, kochte kurz mit etwas Aktivkohle, filtrierte diese heiß ab, ließ abkühlen, saugte den gebildeten Niederschlag ab und trocknete am Rotationsverdampfer.

Ausbeute 0,1 g (32% d.Th.) logP (HCOOH) 0,95

1H-NMR (d6-DMSO): 3,1(s, 3H, unter DMSO), 3,4 (2, 3H), 7,4 (m, 1H), 8,4 (m, 1H), 8,8 (m, 3H), 9,2 (m, 1H)

In der folgenden Tabelle sind weitere erfindungsgemäße Verbindungen aufgeführt.

**Tabelle 1 (NMR-Daten für einige Verbindungen sind in der nachfolgenden Tabelle 2 aufgeführt) h n**

| Verbindung Nr. | **Formel** | logP¹⁾ (HCOOH) | [M+1] aus LC/MS |
|---|---|---|---|
| Herstellungs beispiel 1 | 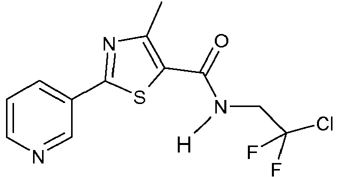 | 1,84 | 318,1 |
| Herstellungs beispiel 2 | 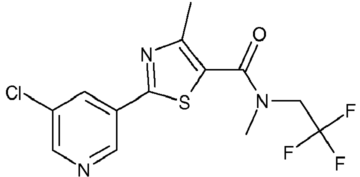 | 2,72 | 350,0 |
| Herstellungs beispiel 3 | 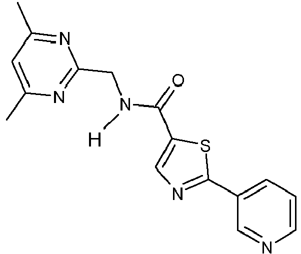 | 1,19 | 326,1 |
| Herstellungs beispiel 4 | 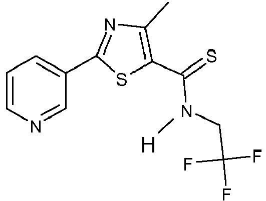 | 2,38 | 318,0 |
| Herstellungs beispiel 5 | 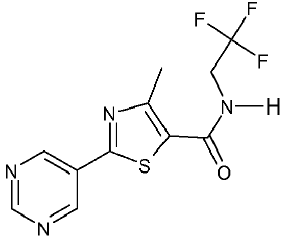 | 1,61 | 289,1 |
| Herstellungs beispiel 6 | 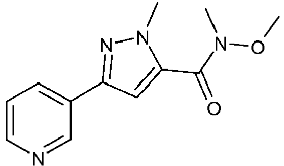 | 0,49 | 247,1 |
| Herstellungs beispiel 7 | 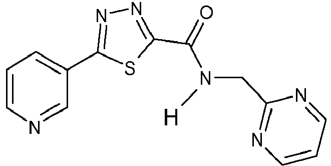 | 0,85 | 299,1 |
| Herstellungs beispiel 8 | 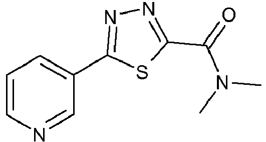 | 0,95 | 235,1 |
| 9 | 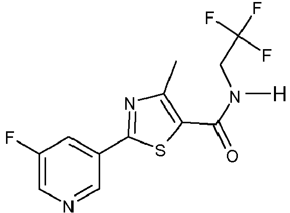 | 2,15 | 320,1 |
| 10 | 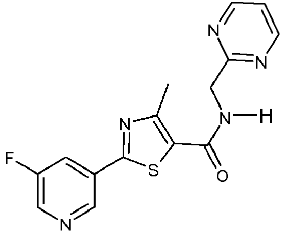 | 1,39 | |
| 11 | 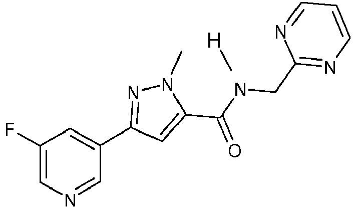 | 1,24 | |
| 12 | 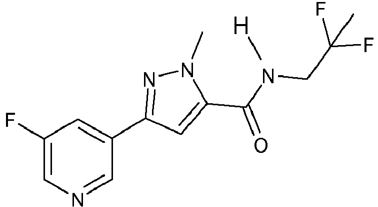 | 1,89 | |
| 13 | 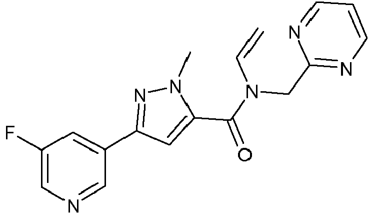 | 1,75 | |
| 14 | 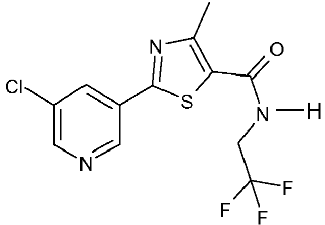 | 2,50 | |
| 15 | 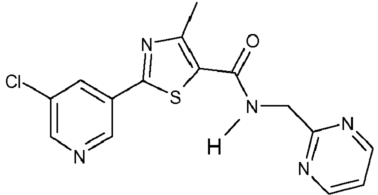 | 1,69 | |
| 16 | 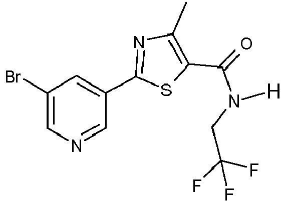 | 2,61 | |
| 17 | 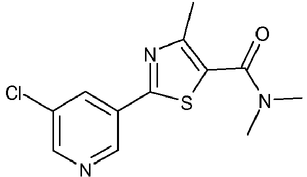 | 1,80 | |
| 18 | 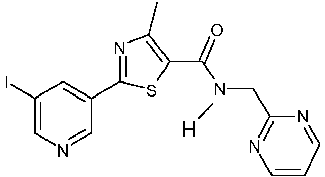 | 1,87 | |
| 19 | 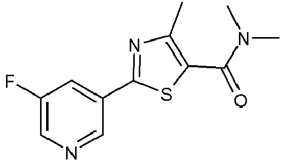 | 1,49 | |
| 20 | 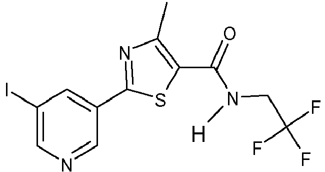 | 2,75 | |
| 21 | 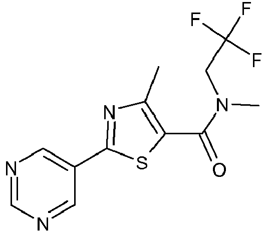 | 1,72 | |
| 22 | 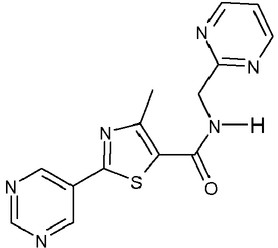 | 1,05 | |
| 23 | 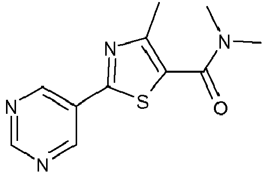 | 1,00 | |
| 24 | 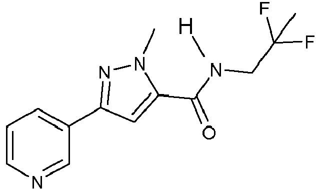 | 0,91 | 281,1 |
| 25 | 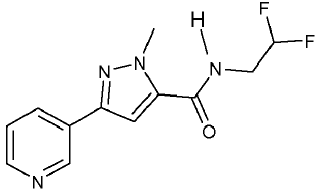 | 0,65 | 267,1 |
| 26 | 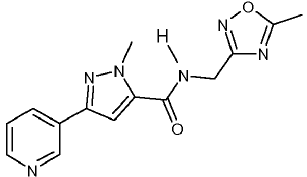 | 0,54 | 299,1 |
| 27 | 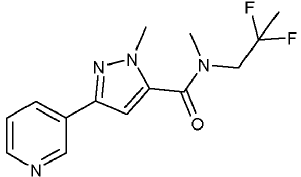 | 0,99 | 295,1 |
| 28 | 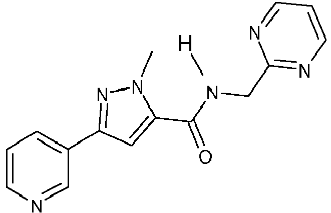 | 0,25 | 295,1 |
| 29 | 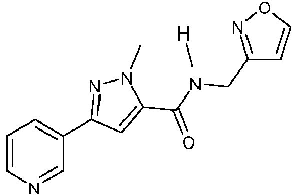 | 0,56 | 284,1 |
| 30 | 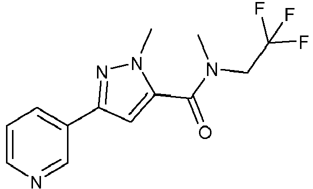 | 1,09 | |
| 31 | 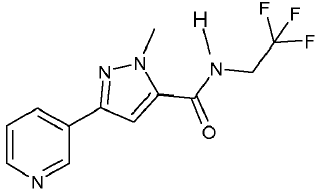 | 1,04 | |
| 32 | 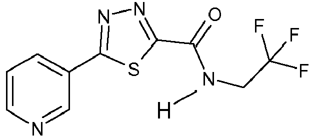 | 1,53 | |
| 33 | 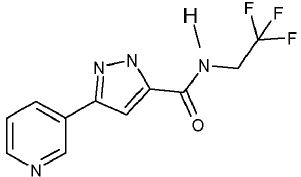 | 0,72 | |
| 34 | 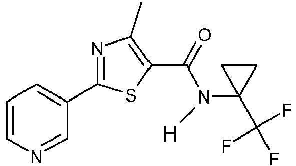 | 1,84 | 328,0 |
| 35 | 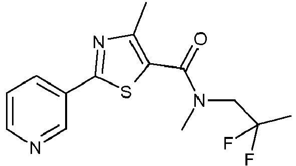 | 1,65 | 312,1 |
| 36 | 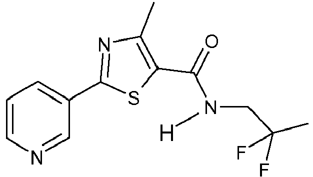 | 1,59 | 298,1 |
| 37 | 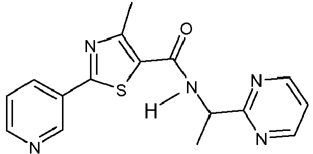 | 1,24 | 326,1 |
| 38 | 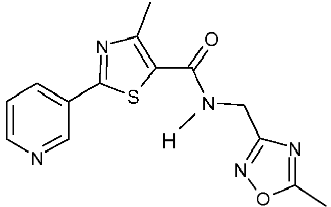 | 1,21 | 316,1 |
| 39 | 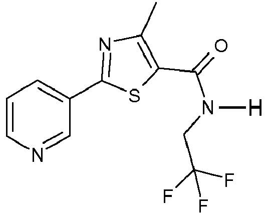 | 1,63 | 302,0 |
| 40 | 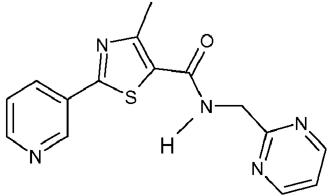 | 1,05 | 312,1 |
| 41 | 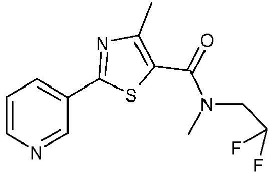 | 1,48 | 298,0 |
| 42 | 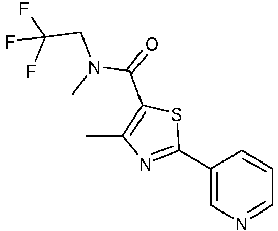 | 1,81 | 316,1 |
| 43 | 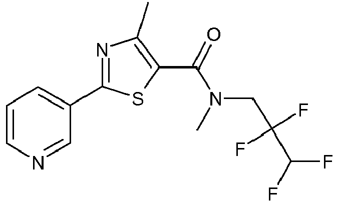 | 1,69 | 348,0 |
| 44 | 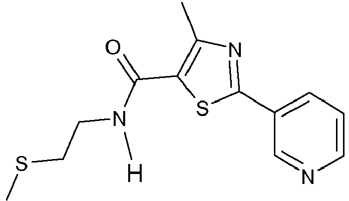 | 1,54 | 293,1 |
| 45 | 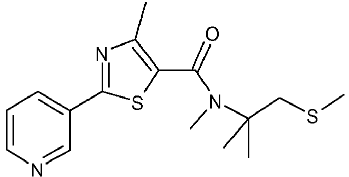 | 2,21 | 336,1 |
| 46 | 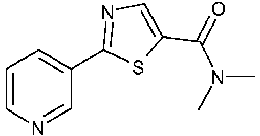 | 0,91 | 234,1 |
| 47 | 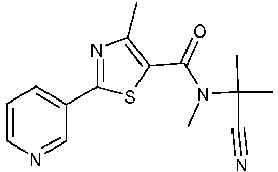 | 1,41 | 301,1 |
| 48 | 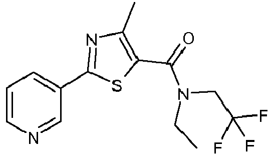 | 2,05 | 330,1 |
| 49 | 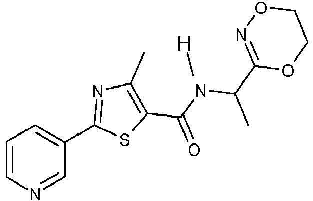 | 1,27 | 333,1 |
| 50 | 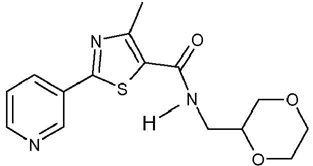 | 0,80 | 320,1 |
| 51 | 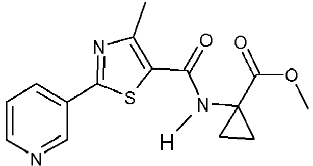 | 1,30 | 318,1 |
| 52 | 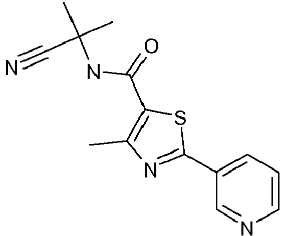 | 1,48 | |
| 53 | 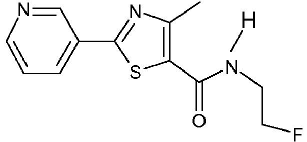 | 1,11 | 266,1 |
| 54 | 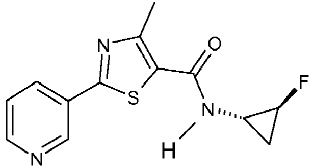 | 1,16 | 278,1 |
| 55 | 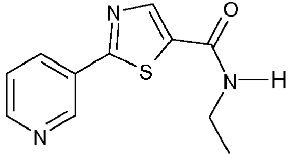 | 1,03 | 234,0 |
| 56 | 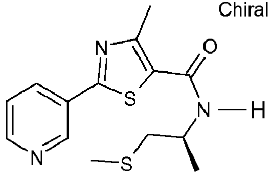 | 1,68 | 307,1 |
| 57 | 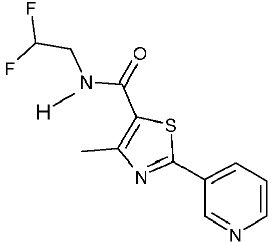 | 1,34 | 284,1 |
| 58 | 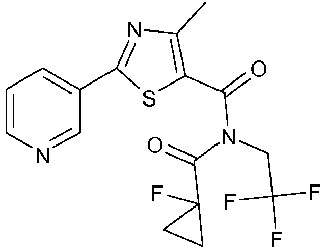 | 2,77 | 388,1 |
| 59 | 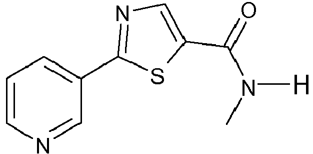 | 0,62 | 220,1 |
| 60 | 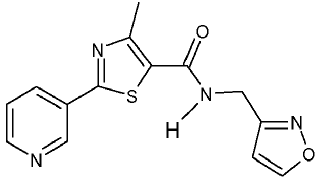 | 1,14 | 301,1 |
| 61 | 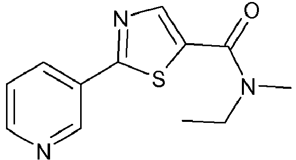 | 1,23 | |
| 62 | 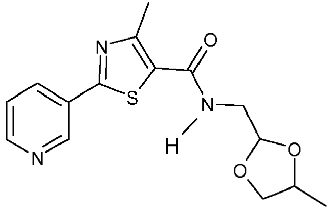 | 1,34 | |
| 63 | 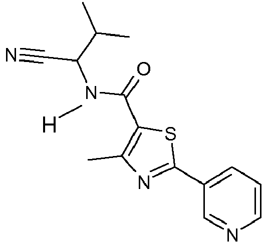 | 1,85 | |
| 64 | 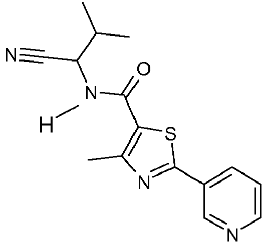 | 1,57 | |
| 65 | 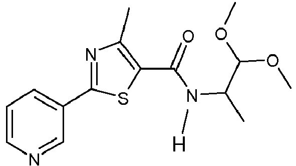 | 1,45 | |
| 66 | 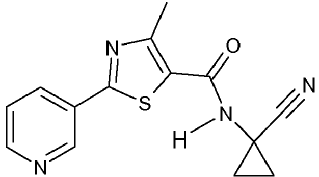 | 1,20 | |
| 67 | 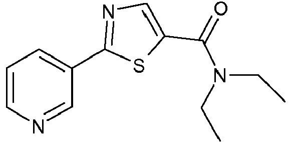 | 1,46 | |
| 68 | 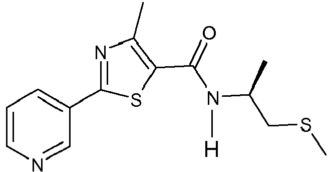 | 1,65 | |
| 69 | 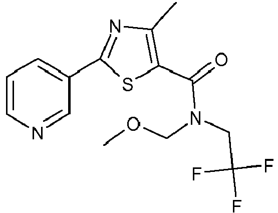 | 2,06 | |
| 70 | 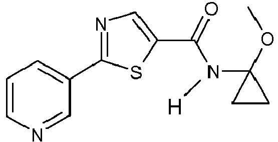 | 1,14 | |
| 71 | 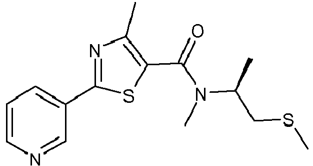 | 1,71 | |
| 72 | 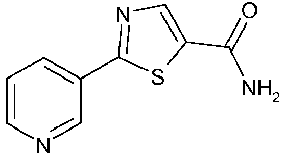 | 0,29 | |
| 73 | 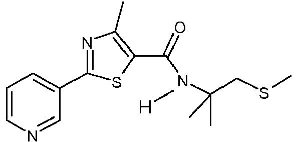 | 2,05 | |
| 74 | 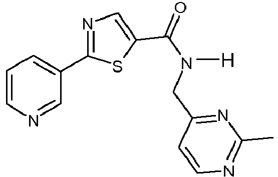 | 0,94 | |
| 75 | 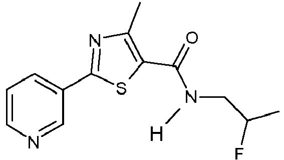 | 1,34 | |
| 76 | 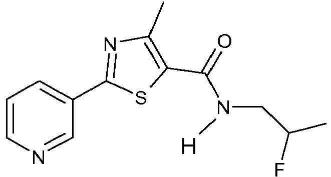 | 1,13 | |
| 77 | 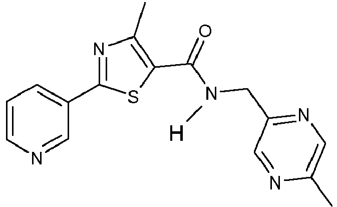 | 1,15 | |
| 78 | 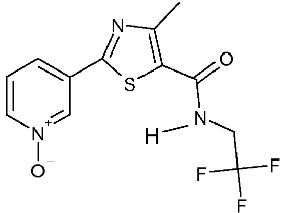 | 1,31 | |
| 79 | 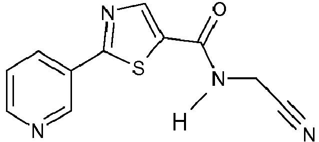 | 0,88 | |
| 80 | 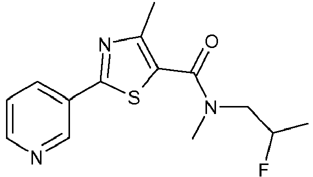 | 1,50 | |
| 81 | 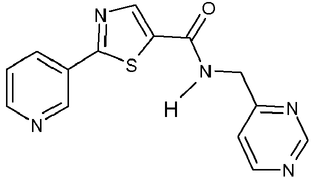 | 0,73 | |
| 82 | 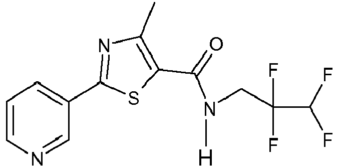 | 1,75 | |
| 83 | 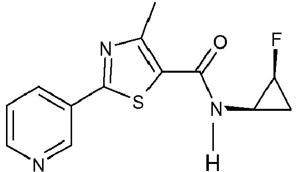 | 1,16 | |
| 84 | 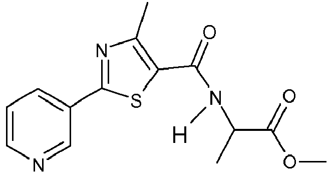 | 1,36 | |
| 85 | 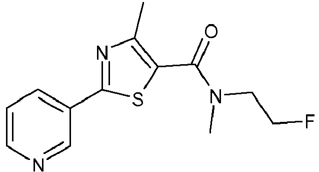 | 1,23 | |
| 86 | 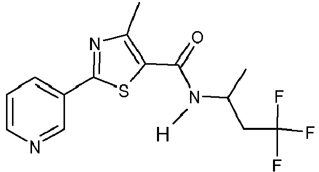 | 1,94 | |
| 87 | 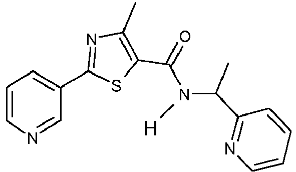 | 1,10 | |
| 88 | 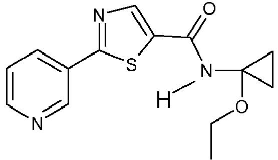 | 1,31 | |
| 89 | 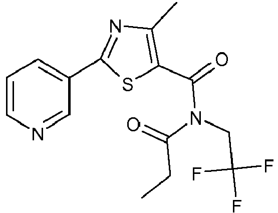 | 2,61 | |
| 90 | 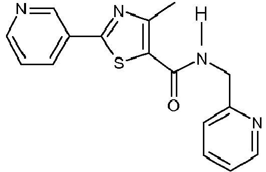 | 0,78 | |
| 91 | 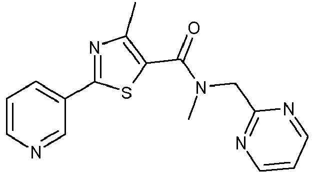 | 1,02 | |
| 92 | 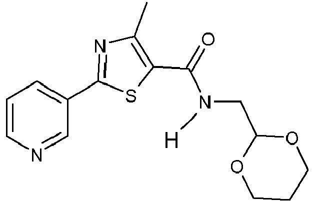 | 1,27 | |
| 93 | 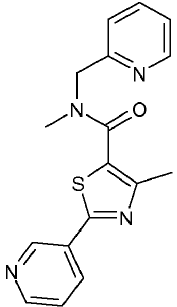 | 1,19 | |
| 94 | 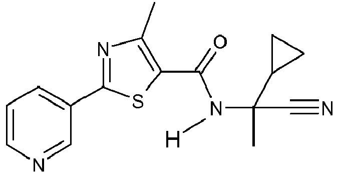 | 1,76 | |
| 95 | 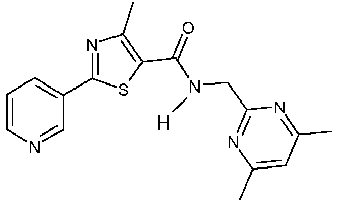 | 1,24 | |
| 96 | 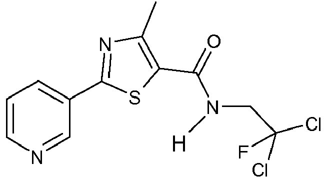 | 1,98 | |
| 97 | 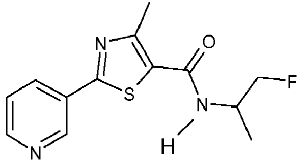 | 1,42 | |
| 98 | 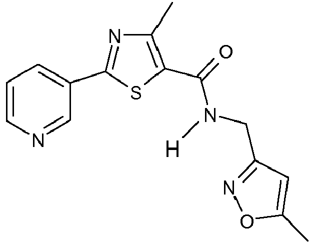 | 1,45 | |
| 99 | 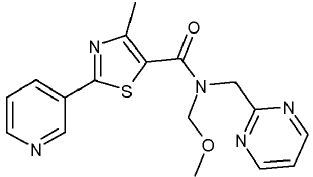 | 1,28 | |
| 100 | 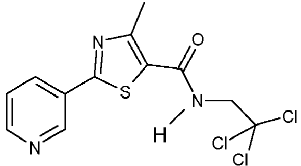 | 2,17 | |
| 101 | 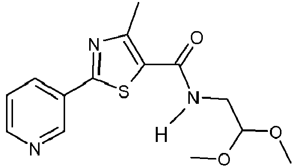 | 1,33 | |
| 102 | 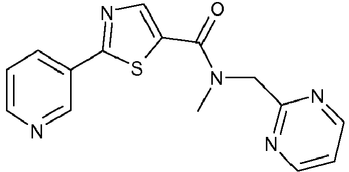 | 1,30 | |
| 103 | 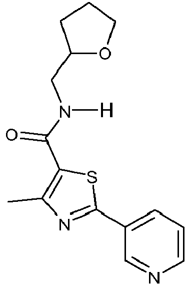 | 1,40 | |
| 104 | 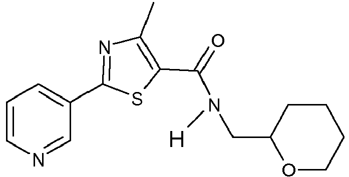 | 1,64 | |
| 105 | 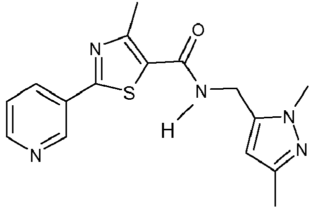 | 1,23 | |
| 106 | 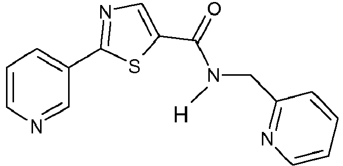 | 0,61 | |
| 107 | 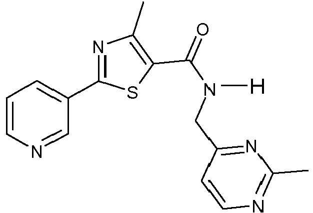 | 1,03 | |
| 108 | 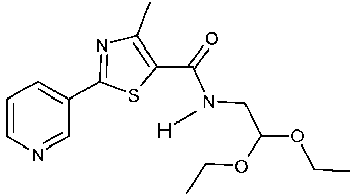 | 1,78 | |
| 109 | 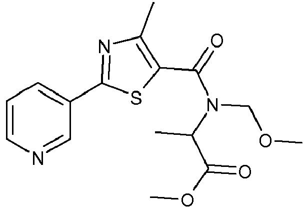 | 1,61 | |
| 110 | 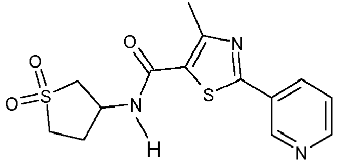 | 1,22 | |
| 111 | 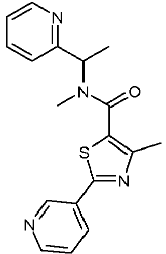 | 1,45 | |
| 112 | 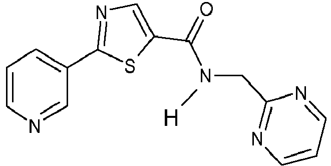 | 0,81 | |
| 113 | 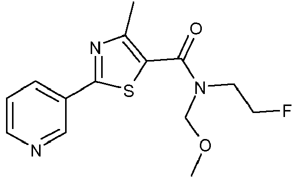 | 1,46 | |
| 114 | 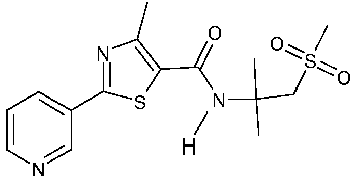 | 1,28 | |
| 115 | 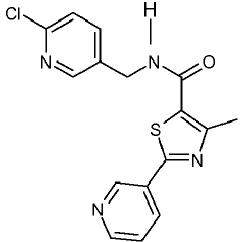 | 1,71 | |
| 116 | 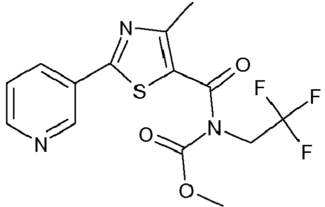 | 2,46 | |
| 117 | 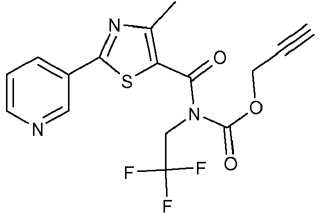 | 2,63 | |
| 118 | 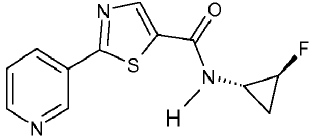 | 1,14 | |
| 119 | 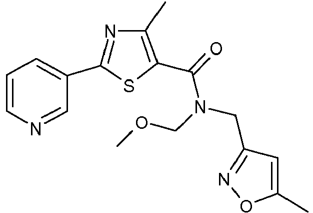 | 1,71 | |
| 120 | 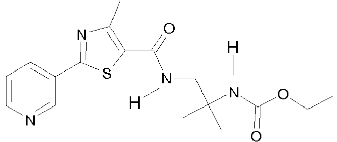 | 1,75 | |
| 121 | 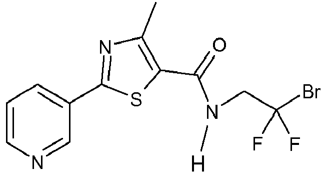 | 1,98 | |
| 122 | 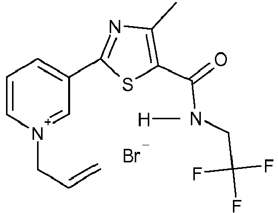 | 1,13 | |
| 123 | 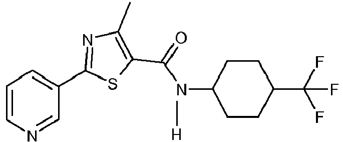 | 2,42 | |
| 124 | 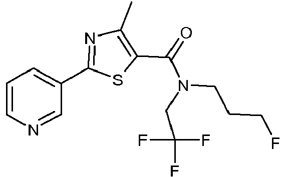 | 2,11 | |
| 125 | 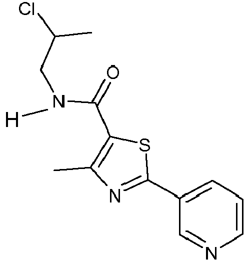 | 1,69 | |
| 126 | 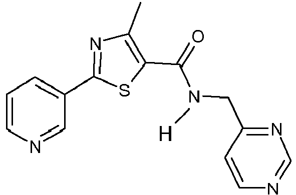 | 0,90 | |
| 127 | 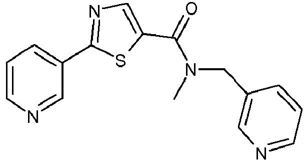 | 0,98 | |
| 128 | 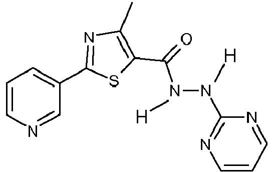 | 0,75 | |
| 129 | 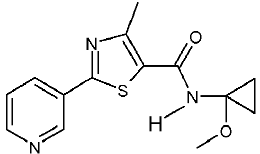 | 1,23 | |
| 130 | 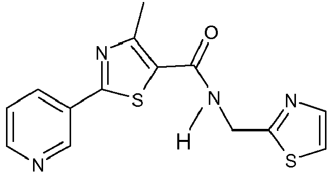 | 1,38 | |
| 131 | 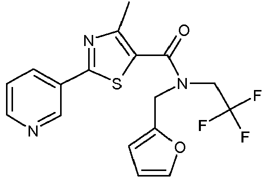 | 2,56 | |
| 132 | 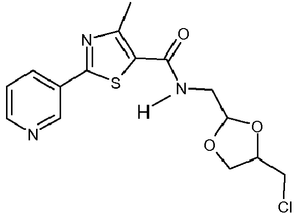 | 1,51 | |
| 133 | 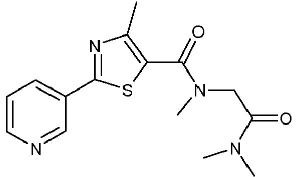 | 0,80 | |
| 134 | 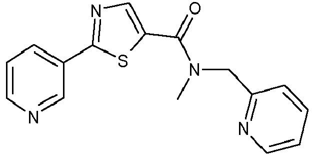 | 0,41 | |
| 135 | 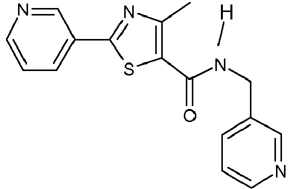 | 0,46 | |
| 136 | 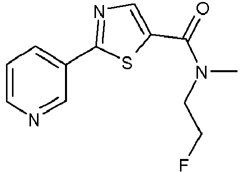 | 1,17 | |
| 137 | 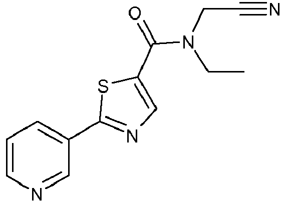 | 1,28 | |
| 138 | 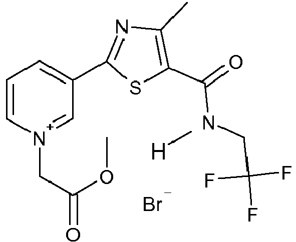 | 1,09 | |
| 139 | 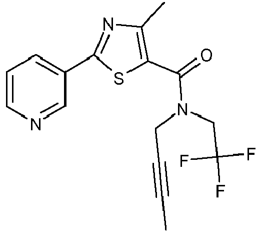 | 2,47 | |
| 140 | 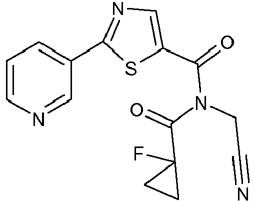 | 1,93 | |
| 141 | 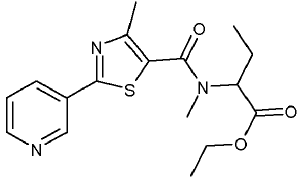 | 2,10 | |
| 142 | 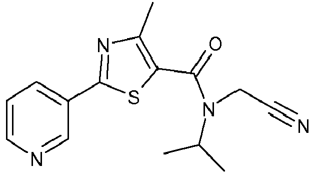 | 1,56 | |
| 143 | 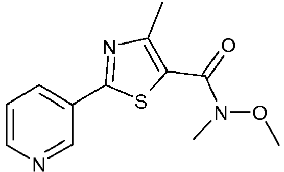 | 1,42 | |
| 144 | 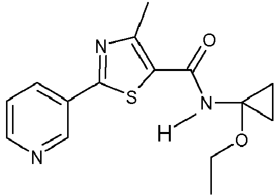 | 1,45 | |
| 145 | 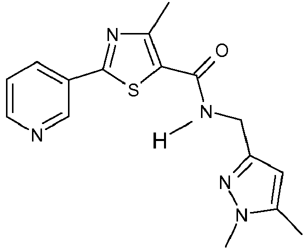 | 1,38 | |
| 146 | 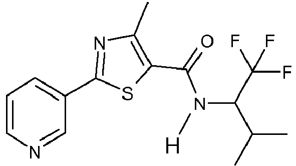 | 2,48 | |
| 147 | 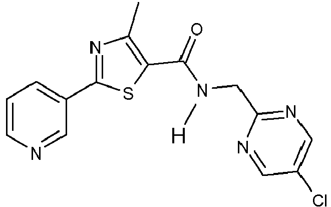 | 1,45 | |
| 148 | 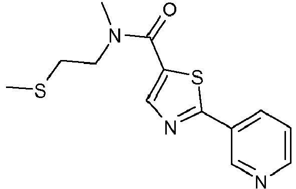 | 1,38 | |
| 149 | 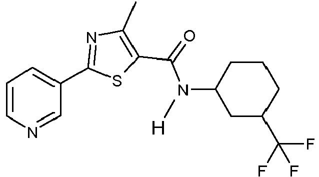 | 2,48 | |
| 150 | 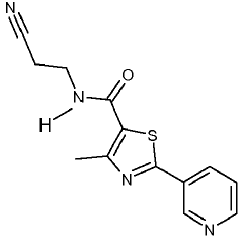 | 1,19 | |
| 151 | 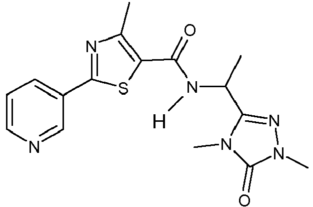 | 1,01 | |
| 152 | 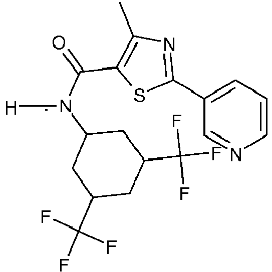 | 2,95 | |
| 153 | 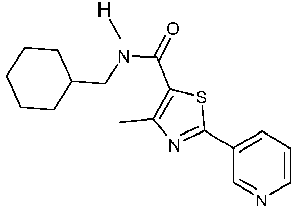 | 2,43 | |
| 154 | 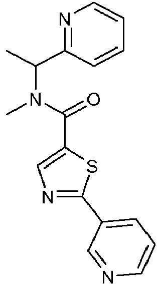 | 1,21 | |
| 155 | 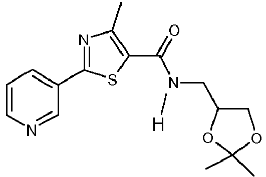 | 1,48 | |
| 156 | 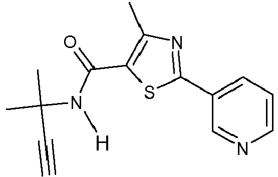 | 1,66 | |
| 157 | 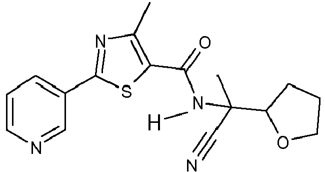 | 1,69 | |
| 158 | 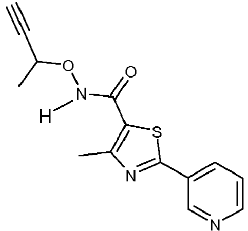 | 1,48 | |
| 159 | 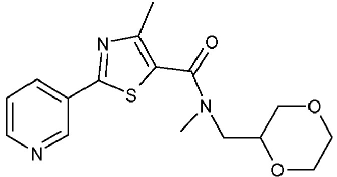 | 1,26 | |
| 160 | 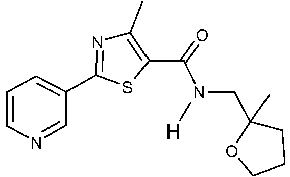 | 1,27 | |
| 161 | 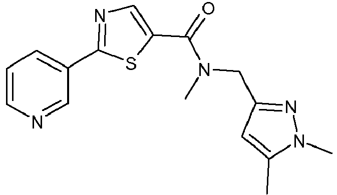 | 1,26 | |
| 162 | 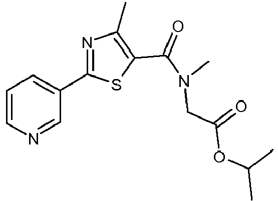 | 1,78 | |
| 163 | 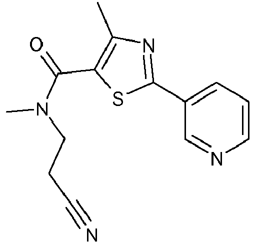 | 1,10 | |
| 164 | 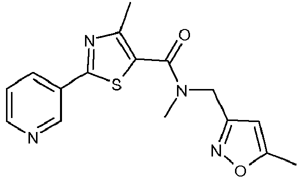 | 1,52 | |
| 165 | 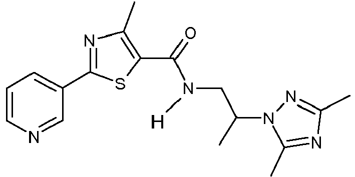 | 1,06 | |
| 166 | 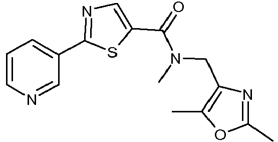 | 1,44 | |
| 167 | 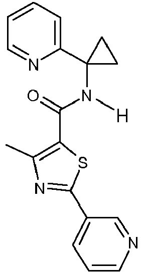 | 0,97 | |
| 168 | 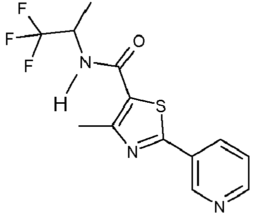 | 1,89 | |
| 169 | 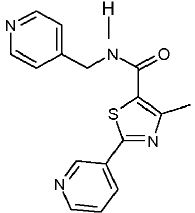 | 0,69 | |
| 170 | 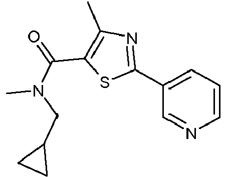 | 1,68 | |
| 171 | 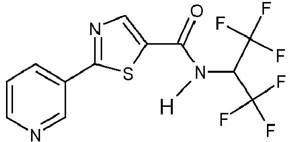 | 2,30 | |
| 172 | 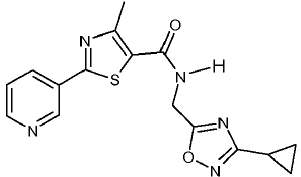 | 1,63 | |
| 173 | 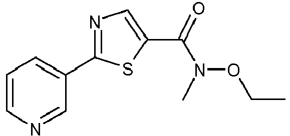 | 1,50 | |
| 174 | 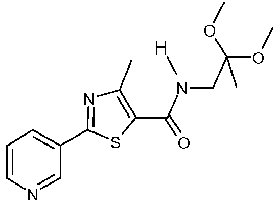 | 1,44 | |
| 175 | 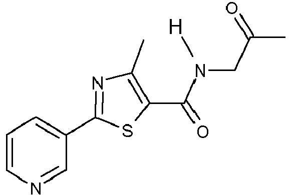 | 1,02 | |
| 176 | 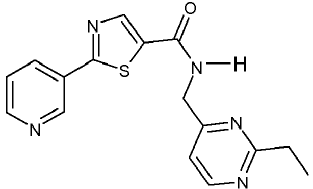 | 1,19 | |
| 177 | 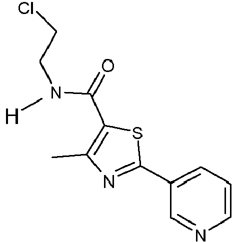 | 1,41 | |
| 178 | 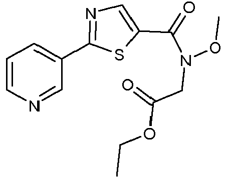 | 1,65 | |
| 179 | 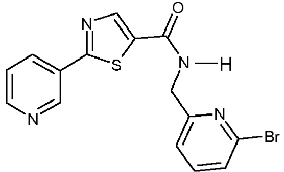 | 1,68 | |
| 180 | 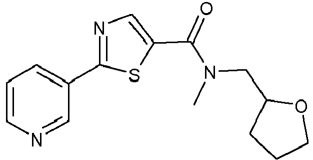 | 1,33 | |
| 181 | 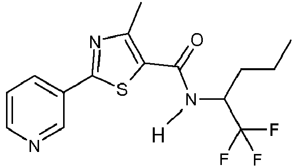 | 2,58 | |
| 182 | 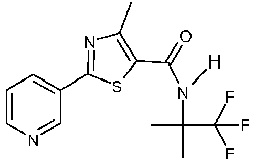 | 2,24 | |
| 183 | 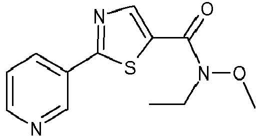 | 1,53 | |
| 184 | 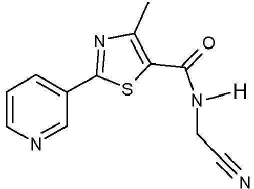 | 1,05 | |
| 185 | 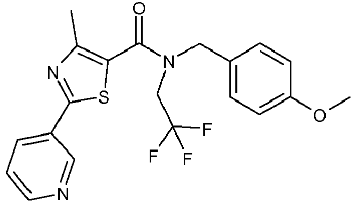 | 2,91 | |
| 186 | 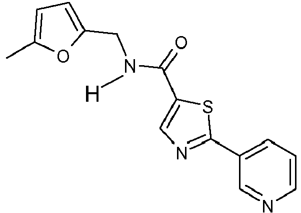 | 1,77 | |
| 187 | 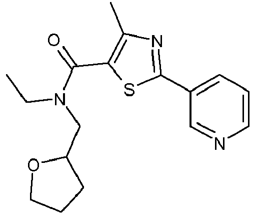 | 1,64 | |
| 188 | 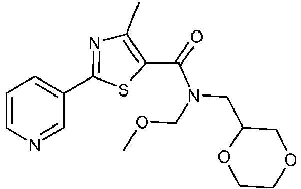 | 1,45 | |
| 189 | 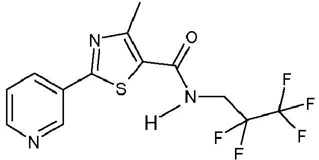 | 2,13 | |
| 190 | 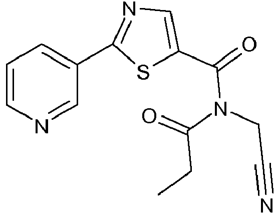 | 1,77 | |
| 191 | 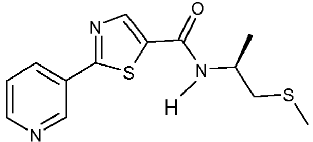 | 1,53 | |
| 192 | 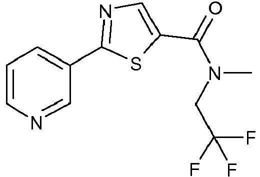 | 1,59 | |
| 193 | 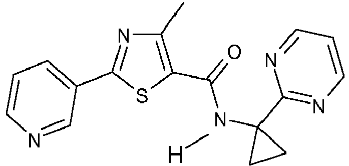 | 1,21 | |
| 194 | 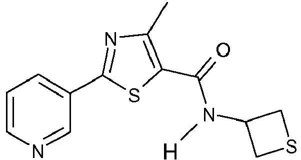 | 1,47 | |
| 195 | 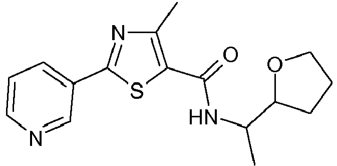 | 1,50 | |
| 196 | 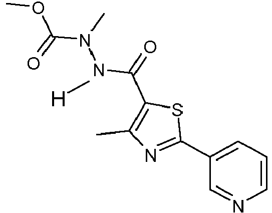 | 1,23 | |
| 197 | 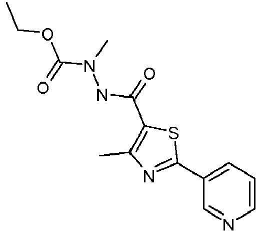 | 1,48 | |
| 198 | 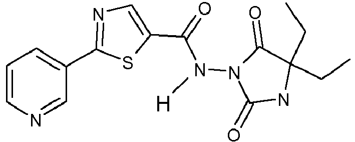 | 1,31 | 338,0 |
| 199 | 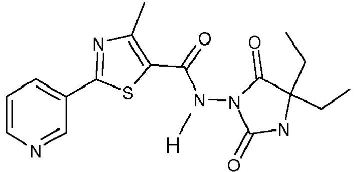 | 1,44 | |
| 200 | 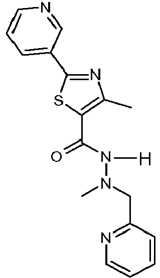 | 1,01 | |
| 201 | 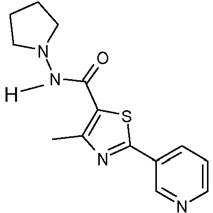 | 1,37 | |
| 202 | 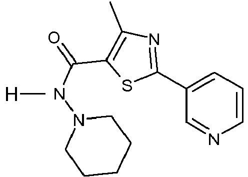 | 1,39 | |
| 203 | 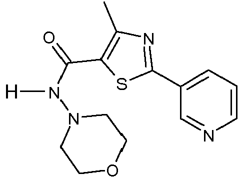 | 1,23 | |
| 204 | 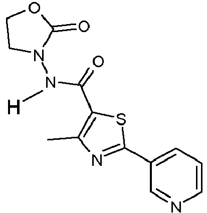 | 1,11 | |

**Tabelle 2**

| Verbindung Nr. | NMR Daten, chem. Verschiebung in ppm. |
|---|---|
| 1 | CD₃CN: 2,7 (s, 3H) 4,2 (dt, 2H), 7,1 (br, 1H), 7,45 (dd, 1H), 8,2 (m, 1H), 8,75 (m, 1H), 9,1 (m, 1H) |
| 2 | d₆-DMSO: 2,43 (s, 3H), 3,13 (s, 3H), 4,35 (q, 2H), 8,37 (m, 1H), 8,73 (m,1H), 9,05 (m,1H) |
| 3 | d₆-DMSO: 2,40 (s, 6H), 4,58 (m, 2H), 7,1 (m, 1H), 7,55 (m, 1H), 8,35 (m, 1H), 8,55 (s, 1H), 8,70 (m, 1H), 9,10 (m, 1H), 9,15 (m, 1H) |
| 4 | d₆-DMSO: 2,54 (s, 3H), 4,65 (q, 2H), 7,50 (m, 1H), 8,25 (m, 1H), 8,65 (m, 1H), 9,10 (m, 1H) |
| 5 | d₆-DMSO: 2,60 (s, 3H), 4,05 (m, 2H), 8,85 (m, 1H), 9,25 (m, 2H) |
| 6 | d₆-DMSO: 3,31 (s, 3H), 3,70 (s, 3H), 4,02 (s, 3H), 7,20 (s, 1H), 7,40 (m, 1H), 8,15 (m, 1H), 8,50 (m, 1H), 9,02 (m, 1H) |
| 7 | d₆-DMSO: 4,25 (m, 2H), 7,4 (t, 1H), 7,6 (m, 1H), 8,4 (m, 1H), 8,8 (m, 3H), 9,2 (m, 1H), 9,4 (br, 1H) |
| 8 | d₆-DMS0:3,1 (s, 3H, unter DMSO), 3,4 (s, 3H), 7,4 (m, 1H), 8,4 (m, 1H), 8,8 (m, 1H), 9,2 (m, 1H) |
| 24 | d₆-DMSO: 1,65 (t, 3H), 3,7 (m, 2H), 4,11 (s, 3H), 7,4 (m, 2H), 8,1 (m, 1H), 8,5 (m, 1H), 8,8 (m, 1H), 8,95 (m, 1H) |
| 25 | d₆-DMSO: 3,7 (m, 2H), 4,11 (s, 3H), 6,11 (m, 1H), 7,45 (m, 1H), 8,1 (m, 1H), 8,5 (m, 1H), 8,8 (m, 1H), 8,95 (m, 1H) |
| 26 | d₆-DMSO: 2,57 (s, 3H), 4,1 (s, 3H), 4,55 (m, 2H), 7,48 (m, 1H), 8,1 (m, 1H), 8,54 (m, 1H), 8,95 (m, 1H), 9,05 (m, 1H) |
| 28 | d₆-DMSO: 4,1 (s, 3H), 4,65 (s, 2H), 7,4 (m, 2H), 8,1 (m, 1H), 8,5 (m, 1H), 8,8-9,0 (m, 4H) |
| 29 | d₆-DMSO: 4,1 (s, 3H), 4,65 (m, 2H), 7,3 (m, 1H), 7,45 (m, 1H), 8,1 (m, 1H), 8,50 (m, 1H), 8,95 (m, 1H), 9,02 (m, 1H) |
| 34 | d₆-DMSO: 1,15 (m, 2H), 1,30 (m, 2H), 2.61 (s, 3H), 7,50 (m, 1H), 8,26 (m,1H), 8,70 (m, 1H), 8,9 (br, 1H), 9,15 (m, 1H) |
| 35 | CD₃CN: 1,6 (t, 3H), 2,45 (s, 3H), 3,1 (s, 3H), 3,9 (t, 2H), 7,45 (m, 1H), 8,25 (d, 1H), 8,65 (m, 1H), 9,1 (s, 1H) |
| 36 | CD₃CN: 1,7 (t, 3H), 2,7 (s, 3H), 3,8 (td, 2H), 6,8 (br, 1H), 7,45 (m, 1H), 8,2 (d, 1H), 8,65 (m, 1H), 9,1 (s, 1H) |
| 37 | d₆-DMSO: 1,55 (d, 3H), 2,55 (s, 3H), 5,2 (m, 1H), 7,38 (m, 1H), 7,52 (m, 1H), 8,25 (m, 1H), 8,50 (m, 1H), 8,7 (m, 1H), 8,75 (m, 2H), 9,1 (m, 1H) |
| 38 | d₆-DMSO: 2,58 (s, 3H), 2,63 (s, 3H), 4,52 (s, 2H), 7,50 (m, 1H), 8,28 (m, 1H), 8,65 (m, 1H), 8,80 (br, 1H), 9,1 (m, 1H) |
| 39 | CD₃CN: 2,7 (s, 3H), 4,1 (m, 2H), 7,05 (br, 1H), 7,45 (m, 1H), 8,25 (m, 1H), 8,7 (m, 1H), 9,15 (s, 1H) |
| 40 | d₆-DMSO: 2,65 (s, 3H), 4,65 (d, 2H), 7,4 (t, 1H), 7,6 (m, 1H), 8,3 (m, 1H), 8,75 (m, 1H), 8,8 (m, 2H), 8,85 (m, 1H), 9,1 (s, 1H) |
| 41 | d₆-DMSO: 2,4 (s, 3H), 3,1 (s, 3H), 3,9 (t, 2H), 6,3 (t, 1H), 7,5 (m, 1H), 8,3 (d, 1H), 8,65 (m, 1H), 9,1 (s, 1H) |
| 42 | d₆-DMSO: 2,45 (s, 3H), 3,15 (s, 3H), 4,35 (q, 2H), 7,65 (m, 1H), 8,3 (d, 1H), 8,7 (d, 1H), 9,1 (s, 1H) |
| 43 | d₆-DMSO: 2,45 (s, 3H), 3,15 (s, 3H), 4,20 (m, 2H), 7,50 (m, 1H), 8,25 (m, 1H), 8,25 (m, 1H), 8,66 (m, 1H), 9,09 (m, 1H) |
| 45 | CD₃CN: 1,50 (s, 6H), 2,15 (s, 3H), 2,5 (s, 3H), 3,02 (s, 3H), 3,25 (s, 2H), 7,45 (m, 1H), 8,20 (m, 1H), 8,61 (m, 1H), 9,1 (m, 1H) |
| 46 | CD₃CN:3,1 (s, 6H), 7,45 (m, 1H), 8,1 (s, 1H), 8,25 (d, 1H), 8,65 (m, 1H), 9,15 (s, 1H) |
| 47 | CD₃CN: 1,78 (s, 6H), 2,49 (s, 3H), 3,0 (s, 3H), 7,45 (m, 1H), 8,20 (m, 1H), 8,65 (m, 1H), 9,1 (m, 1H) |

| Verbindung Nr. | NMR Daten |
|---|---|
| 48 | CD₃CN: 1,1 (t, 3H), 2,43 (s, 3H), 3,5 (m, 2H), 4,22 (m, 2H), 7,45 (m, 1H), 8,22 (m, 1H), 8,65 (m, 1H), 9,1 (m, 1H) |
| 49 | d₆-DMSO: 1,35 (d, 3H), 2,60 (s, 3H), 4,0 (m, 2H), 4,32 (m, 2H), 4,58 (m, 1H), 7,50 (m, 1H), 8,25 (m, 1H), 8,30 (m, 1H), 8,65 (m, 1H), 9,15 (m, 1H) |
| 50 | d₆-DMSO: 2,60 (s, 3H), 3,23-3,80 (m, 9H), 4,75 (m, 1H), 7,50 (m, 1H), 8,15 (m, 1H), 8,25 (m, 1H), 8,70 (m, 1H), 9,08 (m, 1H) |
| 51 | d₆-DMSO: 1,18 (m, 2H), 1,45 (m, 2H), 2,60 (s, 3H), 7,55 (m, 1H), 8,25 (m, 1H), 8,65 (m, 1H), 8,80 (br, 1H), 9,15 (m, 1H) |
| 53 | CD₃CN: 2,7 (s, 3H), 3,7 (dq, 2H), 4,6 (dt, 2H), 6,8 (br, 1H), 7,45 (m, 1H), 8,2 (d, 1H), 8,65 (d, 1H), 9,1 (s, 1H) |
| 54 | d₆-DMSO: 1,1-1,2 (m, 2H), 2,50 (s, 3H), 2,80 (m, 1H), 4,75 (m, 1H), 7,55 (m, 1H), 8,25 (m, 1H), 8,30 (m, 1H), 8,65 (m, 1H), 9,11 (m, 1H) |
| 55 | CD₃CN:1,2 (t, 3H), 3,35 (m, 2H), 7 (br, 1H), 7,45 (m, 1H), 8,2 (s, 1H), 8,25 (d, 1H), 8,65 (m, 1H), 9,1 (s, 1H) |
| 57 | CD₃CN: 2,7 (s, 3H), 3,7 (m, 2H), 6 (t, 1H), 6,9 (br, 1H), 7,45 (m, 1H), 8,25 (d, 1H), 8,65 (m, 1H), 9,1 (s, 1H) |
| 58 | d₆-DMSO: 1,2-1,50 (m, 4H), 2,46 (s, 3H), 4,65 (m, 2H), 7,55 (m, 1H), 8,34 (m, 1H), 8,71 (m, 1H), 9,15 (m, 1H) |
| 59 | CD₃CN: 2,9 (m, 3H), 6,9 (br, 1H), m 7,45 (m, 1H), 8,2 (s, 1H), 8,25 (d, 1H), 8,7 (m, 1H), 9,1 (s, 1H) |
| 60 | d₆-DMSO: 2,62 (s, 3H), 4,55 (s, 2H), 6,50 (s, 1H), 7,55 (m, 1H), 8,29 (m,1H), 8,69 (m, 1H), 8,75 (br, 1H), 8,80 (m, 1H), 9,09 (m, 1H) |
| 198 | d₆-DMSO: 1,37-1,40 (m, 2H), 1,60-1,62 (m, 2H), 2,67 (s, 3H), 7,25 (t, 1H), 7,52-7,56 (m, 1H), 8,26-8,29 (m, 1H), 8,67-8,69 (m, 3H), 8,83 (bs, 1H), 9,11 (d, 1H) |

### 1) Methodenbeschreibung zur Bestimmung der logP Werte (Ameisensäure Methode)

Die Bestimmung der in der Tabelle angegebenen logP-Werte erfolgte gemäß EEC-Directive 79/831 Annex V.A8 durch HPLC (High Performance Liquid Chromato-graphy) an einer Phasenumkehrsäule (C 18). Temperatur: 55°C.

Eluenten für die Bestimmung im sauren Bereich (pH 3,4):
Eluenat A: Acetonitril + 1 ml Ameisensäure/Liter. Eluent B: Wasser + 0,9 ml Ameisensäure/Liter.
Gradient: von 10% Eluent A / 90% Eluent B bis 95% Eluent A / 5% Eluent B in 4,25 min.

Die Eichung erfolgte mit unverzweigten Alkan-2-onen (mit 3 bis 16 Kohlen-stoff atomen), deren logP-Werte bekannt sind (Bestimmung der logP-Werte anhand der Retentionszeiten durch lineare Interpolation zwischen zwei aufeinander folgenden Alkanonen). Die lambda-max-Werte wurden an Hand der UV-Spektren von 200 nm bis 400 nm in den Maxima der chromatographischen Signale ermittelt.

### Biologische Beispiele

### Beispiel Nr. 1

### Myzus-Test

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis),* die von allen Stadien der Grünen Pfirsichblattlaus (*Myzus persicae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 70 % bei einer Aufwandmenge von 500 g/ha:
Bsp. Nr. 1, 3, 4, 5, 7, 8, 9, 10, 11, 12, 13, 14, 15, 16, 21, 22, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129,130,131,132,133,134,135,136,137,138,139,140,141,142,143,144,145,146,147,148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204

### Beispiel Nr. 2

### Phaedon-Test

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele eine Wirkung von ≥ 80 % bei einer Aufwandmenge von 500 g/ha:
Bsp. Nr. 39, 75

### Beispiel Nr. 3

### Spodoptera frugiperda-Test

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele eine Wirkung von ≥ 80 % bei einer Aufwandmenge von 500 g/ha:

### Example No. 39

### Beispiel Nr. 4

### Tetranychus-Test, OP-resistent

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von ≥ 80 % bei einer Aufwandmenge von 100 g/ha:
Bsp. Nr. 53

### Beispiel Nr. 5

### Meloidogyne-Test

| | |
|---|---|
| Lösungsmittel: | 80 Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, *Meloidogyne incognita*-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigen z. B. die folgenden Verbindungen der Herstellungsbeispiele eine Wirkung von ≥ 80 % bei einer Aufwandmenge von 20 ppm:
Bsp. Nr. 98, 200

### Beispiel Nr. 6

### Boophilus microplus -Test

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier.

Nach der gewünschten Zeit wird die Wirkung in % bestimmt. Dabei bedeutet 100%, dass keine Zecke fertile Eier gelegt hat.

Bei diesem Test zeigen z. B. die folgenden Verbindungen der Herstellungsbeispiele eine Wirkung von ≥ 80 % bei einer Aufwandmenge von 20 µg/Tier:
Bsp. Nr. 35, 53, 67, 70, 85, 87, 111

### Beispiel Nr. 7

### Lucilia cuprina-Test

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele eine Wirkung von ≥ 80 % bei einer Aufwandmenge von 100 ppm:
Bsp. Nr. 35

## Patentansprüche

1. Verbindungen der Formel (I) 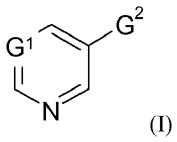 worin
(Ia)
G¹ für N oder C-Halogen steht, und
G² für 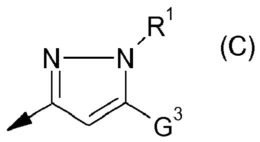
steht,
worin
R¹ für Wasserstoff oder Alkyl steht und
G³ für C(=X)NR²R³ steht,
worin
X für Sauerstoff oder Schwefel steht,
R² für einen Rest aus der Reihe Wasserstoff, Alkyl, Halogenalkyl, Alkoxy, Halogenalkoxy, Alkenyl, Alkoxyalkyl, gegebenenfalls durch Halogen substituiertes Alkylcarbonyl, gegebenenfalls durch Halogen substituiertes Alkoxycarbonyl und gegebenenfalls durch Halogen substituiertes Cycloalkylcarbonyl steht,
und
R³ für einen Rest aus der Reihe Wasserstoff, Alkyl, Halogenalkyl, Cyanoalkyl, Alkenyl, Halogenalkenyl, Alkinyl, Halogenalkinyl, Alkoxy, Halogenalkoxy, gegebenenfalls durch Halogen substituiertes Alkoxyalkyl, gegebenenfalls durch Halogen substituiertes Bis(alkoxy)alkyl, gegebenenfalls durch Halogen substituiertes Alkylthioalkyl, gegebenenfalls durch Halogen substituiertes Alkylcarbonylalkyl, gegebenenfalls durch Halogen substituiertes Alkylsulfinylalkyl, gegebenenfalls durch Halogen substituiertes Alkylsulfonylalkyl, gegebenenfalls durch Halogen substituiertes Alkoxycarbonylalkyl, Alkinyloxy, Alkinyloxycarbonyl, gegebenenfalls substituiertes Cycloalkyl, gegebenenfalls substituiertes Cycloalkylcarbonyl, gegebenenfalls substituiertes Cycloalkylalkyl, gegebenenfalls substituiertes Heterocyclyl, gegebenenfalls substituiertes Heterocyclylalkyl, gegebenenfalls substituiertes Arylalkyl, gegebenenfalls substituiertes Hetarylalkyl und NR⁴R⁵ steht, worin R⁴ und R⁵ unabhängig voneinander für einen Rest aus der Reihe Wasserstoff, Alkyl, Halogenalkyl, Cycloalkyl, Alkoxy, Alkylcarbonyl, Alkoxycarbonyl, Hetaryl und Heterocyclyl stehen oder R⁴ und R⁵ gemeinsam mit dem Stickstoffatom, an das sie gebunden sind, einen gegebenenfalls substituierten Hetrocyclus bilden,
oder
R² und R³ gemeinsam mit dem Stickstoffatom, an das sie gebunden sind, einen gegebenenfalls substituierten und gegebenenfalls weitere Heteroatome enthaltenden Ring bilden,
oder
(Ib)
worin
G¹ für CH steht und
G² für 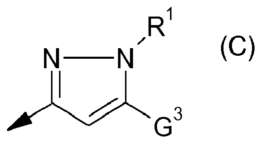
steht, worin
R¹ für Wasserstoff oder Alkyl steht und
G³ für C(=X)NR²R³ steht,
worin
X für Sauerstoff oder Schwefel steht,
R² die oben angegebenen Bedeutungen hat und
R³ die oben angegebenen Bedeutungen hat oder
R² und R³ gemeinsam mit dem Stickstoffatom, an das sie gebunden sind, einen gegebenen-falls substituierten und gegebenenfalls weitere Heteroatome enthaltenden Ring bil-den,
sowie Salze und N-Oxide der Verbindungen der Formel (I).

2. Mittel, **gekennzeichnet durch** einen Gehalt von mindestens einer Verbindung der Formel (I) gemäß Anspruch 1 und üblichen Streckmitteln und/oder oberflächenaktiven Substanzen.

3. Verfahren zum Bekämpfen von Schädlingen **dadurch gekennzeichnet, dass** man eine Verbindung der Formel (I) gemäß Anspruch 1 oder ein Mittel gemäß Anspruch 2 auf die Schädlinge und/oder ihren Lebensraum einwirken lässt.

4. Verwendung von Verbindungen der Formel (I) gemäß Anspruch 1 oder von Mitteln gemäß Anspruch 2 zum Bekämpfen von Schädlingen.
